(19) Europäisches Patentamt / European Patent Office / Office européen des brevets

(11) **EP 4 144 778 A1**

(12) **EUROPEAN PATENT APPLICATION**
published in accordance with Art. 153(4) EPC


(43) Date of publication:
**08.03.2023 Bulletin 2023/10**

(21) Application number: **21796825.4**

(22) Date of filing: **28.04.2021**

(51) International Patent Classification (IPC):
***C08G 18/20*** *(2006.01)* ***C08G 18/80*** *(2006.01)*

(52) Cooperative Patent Classification (CPC):
**C08G 18/20; C08G 18/80**

(86) International application number:
**PCT/JP2021/016895**

(87) International publication number:
**WO 2021/221084 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2020 JP 2020079967**



(71) Applicant: **Koei Chemical Company, Limited**
**Sodegaura-shi, Chiba 299-0266 (JP)**

(72) Inventors:
- **MIYAGI, Motoyoshi**
  **Sodegaura-shi, Chiba 299-0266 (JP)**
- **ONODA, Mitsuki**
  **Sodegaura-shi, Chiba 299-0266 (JP)**
- **INOUE, Naohito**
  **Sodegaura-shi, Chiba 299-0266 (JP)**

(74) Representative: **Müller-Boré & Partner Patentanwälte PartG mbB**
**Friedenheimer Brücke 21**
**80639 München (DE)**


(54) **BLOCKED ISOCYANATE COMPOSITION, HEAT-CURABLE RESIN COMPOSITION, CURED PRODUCT, PRODUCTION METHOD THEREFOR, AMIDATE COMPOUND, AND CATALYST FOR DISSOCIATION OF BLOCKING AGENT FOR BLOCKED ISOCYANATES**


(57) The present invention provides a blocked isocyanate composition comprising a blocked isocyanate compound in which an isocyanate group of an isocyanate compound is blocked with a fluoroalcohol compound, and at least one amidate compound represented by the following Formula (2):

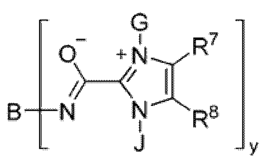
(2)

wherein B, G, J, $R^7$, and $R^8$ are as defined in the specification.


EP 4 144 778 A1

## Description

Technical Field

The present invention relates to a blocked isocyanate composition, a thermosetting resin composition, a cured product and a production method therefor, an amidate compound, and a blocking agent dissociation catalyst for blocked isocyanates.

Background Art

Blocked isocyanate compositions are compositions comprising a blocked isocyanate compound and a blocking agent dissociation catalyst. Blocked isocyanate compounds are compounds obtained by reaction of an isocyanate compound with a blocking agent containing active hydrogen groups that are capable of reacting with isocyanate groups. Blocked isocyanate compounds are inactive at ordinary temperatures with the isocyanate group of the isocyanate compound being blocked by a blocking agent, such as methyl ethyl ketoxime; however, heating causes dissociation of the blocking agent to regenerate the isocyanate group. Blocked isocyanate compounds having such properties have been widely used for applications such as paints and adhesives, and as a raw material or a crosslinking agent for the production of one-component thermosetting polyurethane resin.

In recent years, curing at lower temperatures is required to reduce energy costs and to process resin materials with lower heat resistance.

A catalyst is sometimes added to lower the curing temperature. Commonly known compounds for use as a catalyst include tin compounds, such as dibutyltin dilaurate (hereinafter referred to as "DBTDL").

A thermosetting resin composition comprising the blocked isocyanate compound mentioned above formed by using a blocking agent and a tin compound typically requires a curing temperature of 120°C or higher.

Known blocking agent dissociation catalysts include 1-methyl-3-n-octylimidazolium-2-N-phenylamidate represented by the following structural formula (Patent Literature (PTL) 1).

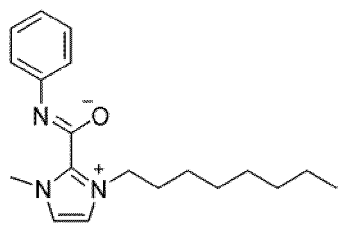

Citation List

Patent Literature

PTL 1: WO2019/065953A1

Summary of Invention

Technical Problem

By using the 1-methyl-3-n-octylimidazolium-2-N-phenylamidate disclosed in PTL 1 as a blocking agent dissociation catalyst, the present inventors evaluated the curing properties of a thermosetting resin composition comprising a blocked polyisocyanate blocked with methyl ethyl ketoxime and a polyol. At 90°C, curing was not observed even after 50 minutes (Comparative Example 4 described below), and the low-temperature curing properties were thus unsatisfactory.

Accordingly, a main object is to provide a blocked isocyanate composition having excellent low-temperature curing properties, as well as a thermosetting resin composition comprising the blocked isocyanate composition, and a cured product thereof.

Solution to Problem

The present invention provides the following blocked isocyanate composition, thermosetting resin composition,

cured product and production method therefor, amidate compound, and blocking agent dissociation catalyst for blocked isocyanates.

[1] A blocked isocyanate composition comprising a blocked isocyanate compound in which an isocyanate group of an isocyanate compound is blocked with a fluoroalcohol compound, and at least one amidate compound represented by the following Formula (2):

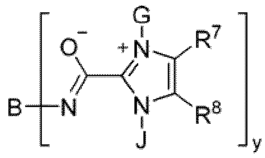 (2)

wherein B represents a substituted or unsubstituted hydrocarbon group; G and J are the same or different, and each represents a $C_1$-$C_{80}$ hydrocarbon group that may be substituted with a heteroatom; $R^7$ and $R^8$ are the same or different, and each represents a hydrogen atom or a $C_1$-$C_{20}$ hydrocarbon group that may be substituted with a heteroatom; G and $R^7$, or $R^8$ and J may form a ring structure together with the nitrogen and carbon atoms to which they are bonded; $R^7$ and $R^8$ may form a ring structure together with the carbon atoms to which they are bonded; and y is an integer of 1 or more and 20 or less.
[2] The blocked isocyanate composition according to [1], wherein the isocyanate compound is at least one polyisocyanate selected from the group consisting of aliphatic polyisocyanates, alicyclic polyisocyanates, aromatic polyisocyanates, and aromatic aliphatic polyisocyanates; or a modified isocyanate formed from at least one member selected from the group consisting of aliphatic polyisocyanates, alicyclic polyisocyanates, aromatic polyisocyanates, and aromatic aliphatic polyisocyanates.
[3] The blocked isocyanate composition according to [1], wherein the isocyanate compound is represented by the following Formula (3):

$$A\left[NCO\right]_x \quad (3)$$

wherein A represents a residue obtained by removing an isocyanate group from at least one polyisocyanate selected from the group consisting of aliphatic polyisocyanates, alicyclic polyisocyanates, aromatic polyisocyanates, and aromatic aliphatic polyisocyanates, or a residue obtained by removing an isocyanate group from a modified isocyanate formed from at least one member selected from the group consisting of aliphatic polyisocyanates, alicyclic polyisocyanates, aromatic polyisocyanates, and aromatic aliphatic polyisocyanates; and x is an integer of 2 or more and 20 or less.
[4] The blocked isocyanate composition according to any one of [1] to [3], wherein the fluoroalcohol compound is represented by Formula (4):

HD-L (4)

wherein L represents a $C_1$-$C_{40}$ fluorine-containing alkyl group.
[5] The blocked isocyanate composition according to any one of [1] to [3], wherein the fluoroalcohol compound is represented by Formula (4a):

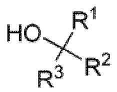 (4a)

wherein $R^1$ and $R^2$ are the same or different, and each represents a hydrogen atom or a $C_1$-$C_8$ fluorine-containing alkyl group; and $R^3$ represents a $C_1$-$C_{20}$ fluorine-containing alkyl group.
[6] The blocked isocyanate composition according to [5], wherein $R^1$ and $R^2$ are the same or different, and each represents a hydrogen atom or a $C_1$-$C_2$ fluorine-containing alkyl group.
[7] The blocked isocyanate composition according to any one of [1] to [6], wherein the amidate compound represented by Formula (2) is an amidate compound represented by Formula (2a):

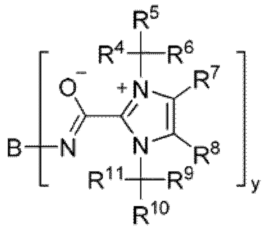 (2a)

wherein B, $R^7$, $R^8$, and y are each as defined above; $R^4$, $R^5$, $R^6$, $R^9$, $R^{10}$, and $R^{11}$ are the same or different, and each represents a $C_1$-$C_{20}$ hydrocarbon group that may be substituted with a heteroatom; $R^4$ and $R^5$, $R^5$ and $R^6$, $R^7$ and $R^8$, $R^9$ and $R^{10}$, or $R^{10}$ and $R^{11}$ may form a ring structure together with the carbon atoms to which they are bonded; and the group represented by $CR^4R^5R^6$ or the group represented by $CR^9R^{10}R^{11}$ may be an adamantyl group.

[8] The blocked isocyanate composition according to any one of [1] to [7], wherein B is a substituted or unsubstituted aromatic hydrocarbon group.

[9] The blocked isocyanate composition according to any one of [1] to [8], wherein $R^7$ and $R^8$ are hydrogen atoms.

[10] The blocked isocyanate composition according to any one of [7] to [9], wherein $R^4$, $R^5$, $R^9$, and $R^{10}$ are the same or different, and each represents a $C_1$-$C_6$ hydrocarbon group that may be substituted with a heteroatom.

[11] The blocked isocyanate composition according to any one of [7] to [10], wherein $R^6$ and $R^{11}$ are the same or different, and each represents a $C_1$-$C_{12}$ hydrocarbon group that may be substituted with a heteroatom.

[12] A thermosetting resin composition comprising the blocked isocyanate composition according to any one of [1] to [11] and a compound having an isocyanate-reactive group.

[13] The thermosetting resin composition according to [12], wherein the compound having an isocyanate-reactive group is a polyol compound.

[14] A cured product obtained by curing the thermosetting resin composition according to [12] or [13].

[15] A method for producing a cured product, comprising curing the thermosetting resin composition according to [12] or [13] by heating.

[16] An amidate compound represented by the following Formula (2a):

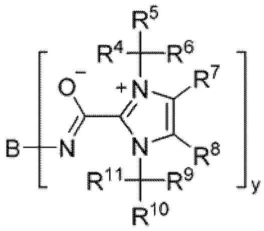 (2a)

wherein B represents a substituted or unsubstituted hydrocarbon group; $R^4$, $R^5$, $R^6$, $R^9$, $R^{10}$, and $R^{11}$ are the same or different, and each represents a $C_1$-$C_{20}$ hydrocarbon group that may be substituted with a heteroatom, or $R^4$ and $R^5$, $R^5$ and $R^6$, $R^9$ and $R^{10}$, or $R^{10}$ and $R^{11}$ may form a ring structure together with the carbon atoms to which they are bonded; $R^7$ and $R^8$ are the same or different, and each represents a hydrogen atom or a $C_1$-$C_{20}$ hydrocarbon group that may be substituted with a heteroatom, or $R^7$ and $R^8$ may form a ring structure together with the carbon atoms to which they are bonded; y is an integer of 1 or more and 20 or less; and the group represented by $CR^4R^5R^6$ or the group represented by $CR^9R^{10}R^{11}$ may be an adamantyl group.

[17] The amidate compound according to [16], wherein $R^4$, $R^5$, $R^9$, and $R^{10}$ are the same or different, and each represents a $C_1$-$C_4$ hydrocarbon group; and $R^6$ and $R^{11}$ are the same or different, and each represents a $C_1$-$C_{12}$ hydrocarbon group.

[18] The amidate compound according to [16], wherein the compound represented by Formula (2a) is any of compounds represented by the following formulas:

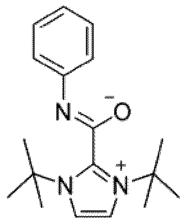,
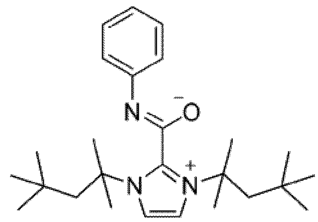,

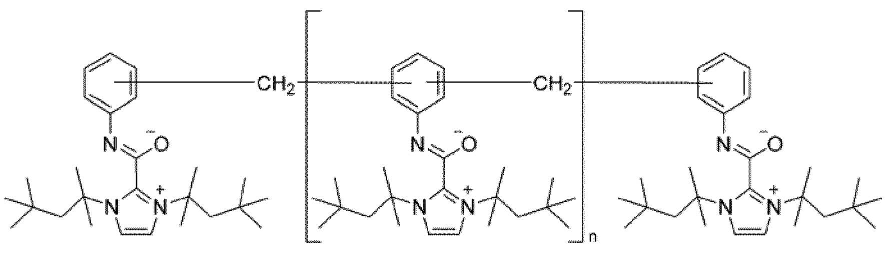,
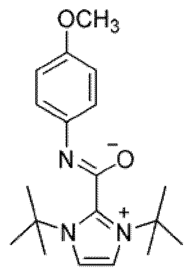,

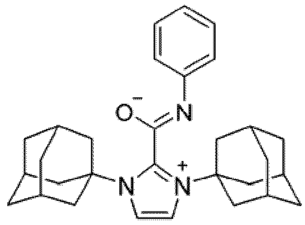

wherein n is 0 or an integer of 1 to 4.

[19] A blocking agent dissociation catalyst for blocked isocyanates comprising the amidate compound according to any one of [16] to [18].

Advantageous Effects of Invention

The present invention can provide a blocked isocyanate composition having excellent low-temperature curing properties, a thermosetting resin composition comprising the blocked isocyanate composition, and a cured product of the thermosetting resin composition.

Description of Embodiments

The blocked isocyanate composition of the present invention comprises a blocked isocyanate compound in which an isocyanate group of an isocyanate compound is blocked with a fluoroalcohol compound (hereinafter referred to as the fluoroalcohol-blocked isocyanate compound (BI)), and at least one amidate compound represented by Formula (2) (hereinafter referred to as the amidate compound (2)).

The fluoroalcohol-blocked isocyanate compound (BI) is a reaction product of an isocyanate compound and a fluoroalcohol compound. The fluoroalcohol-blocked isocyanate compound (BI) may be a compound having, in the molecule, an isocyanate group blocked with at least one fluoroalcohol compound, and preferably a compound having, in the molecule, isocyanate groups blocked with two or more fluoroalcohol compounds.

The fluoroalcohol-blocked isocyanate compound (BI) is more preferably a blocked isocyanate compound represented by Formula (1) (hereinafter also referred to as the blocked polyisocyanate (1)).

Formula (1):

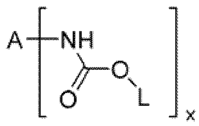 (1)

wherein A is a residue obtained by removing an isocyanate group from at least one polyisocyanate selected from the group consisting of aliphatic polyisocyanates, alicyclic polyisocyanates, aromatic polyisocyanates, and aromatic aliphatic polyisocyanates, or a residue obtained by removing an isocyanate group from a modified isocyanate formed from at least one member selected from the group consisting of aliphatic polyisocyanates, alicyclic polyisocyanates, aromatic polyisocyanates, and aromatic aliphatic polyisocyanates; L represents a $C_{1}$-$C_{40}$ fluorine-containing alkyl group; and x is an integer of 2 or more and 20 or less.

In Formula (1), A represents any of the following residues (i) to (v) (hereinafter also referred to simply as the "residue"):

(i) a residue obtained by removing an isocyanate group from an aliphatic polyisocyanate,
(ii) a residue obtained by removing an isocyanate group from an alicyclic polyisocyanate,
(iii) a residue obtained by removing an isocyanate group from an aromatic polyisocyanate,
(iv) a residue obtained by removing an isocyanate group from an aromatic aliphatic polyisocyanate, and
(v) a residue obtained by removing an isocyanate group from a modified isocyanate formed from at least one member selected from the group consisting of aliphatic polyisocyanates, alicyclic polyisocyanates, aromatic polyisocyanates, and aromatic aliphatic polyisocyanates.

The aliphatic polyisocyanate (i), alicyclic polyisocyanate (ii), aromatic polyisocyanate (iii), aromatic aliphatic polyisocyanate (iv), and modified isocyanate (v) formed from at least one member selected from the group consisting of aliphatic polyisocyanates, alicyclic polyisocyanates, aromatic polyisocyanates, and aromatic aliphatic polyisocyanates are all compounds having an isocyanate group, and the residue A refers to a part obtained by removing one or more isocyanate groups from these compounds. The residue A is generally an x-valent hydrocarbon group that may have a substituent other than isocyanate groups and/or that may be substituted with a heteroatom, and preferably an x-valent hydrocarbon group that may be substituted with a heteroatom and/or a halogen atom. In this case, the number of carbon atoms in the hydrocarbon group is more preferably 1 to 100. x in "x-valent" is the same number as x in Formula (1).

The residue A is an x-valent hydrocarbon group that may have a substituent other than isocyanate groups. Examples of substituents include halogen atoms, such as fluorine, chlorine, bromine, and iodine; dialkylamino groups, alkoxy groups, aryloxy groups, aralkyloxy groups, nitro groups, cyano groups, sulfonyl groups, (monoalkylamino)carbonylamino groups, and (dialkylamino)carbonylamino groups. When the residue A is substituted, the number of substituents is preferably 1 to 5, more preferably 1 to 3, and even more preferably 1 or 2.

Moreover, the hydrocarbon group of the residue A may be substituted with a heteroatom, such as oxygen, nitrogen, or sulfur. When the hydrocarbon group of the residue A is substituted with a heteroatom, such as oxygen, nitrogen, or sulfur, the hydrocarbon group has a group, such as -O-, -N<, -S-, or $-SO_2-$, and the hydrocarbon chain is interrupted by such a group.

Examples of the x-valent hydrocarbon group in the x-valent hydrocarbon group that may have a substituent other than isocyanate groups and/or that may be substituted with a heteroatom include alkylene groups, such as ethylene, n-propylene, n-butylene, n-pentylene, n-hexylene, n-heptylene, n-octylene, n-nonylene, n-decylene, n-dodecylene, n-octadecylene, cyclohexylene, cyclohexane-1,2-diylbismethylene, and cyclohexane-1,4-diylbismethylene; arylene groups, such as p-phenylene, m-phenylene, 2-methyl-m-phenylene, 4-methyl-m-phenylene, 5-methyl-m-phenylene, and naphthylene; arylalkylene groups, such as phenylethylene, 1-phenylpropylene, 2-phenylpropylene, 1-phenylbutylene, 2-phenylbutylene, and naphthylethylene; alkylenearylene groups, such as methylene diphenylene and polymethylene polyphenylene, obtained by suitably combining the above alkylene groups and arylene groups; and the like.

Preferable examples of the residue A include the following groups.

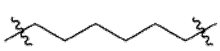, 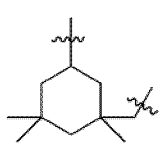, 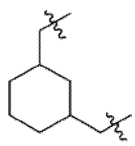, 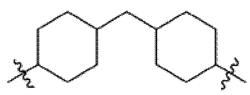

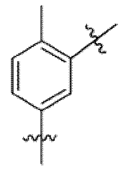, 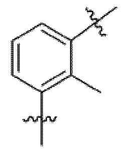, 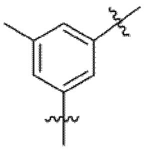, 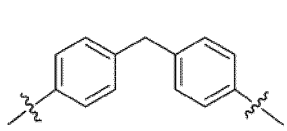,

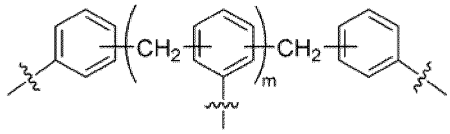,

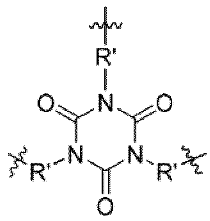, 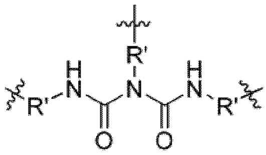, 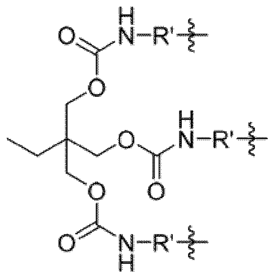

R' = 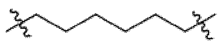, 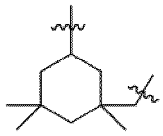, 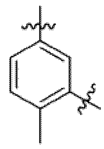

wherein m is an integer of 0 to 4.

x is an integer of 2 or more and 20 or less, preferably 2 to 6, more preferably 2 to 4, and particularly preferably 2 or 3.

L represents a $C_{1\text{-}}C_{40}$ fluorine-containing alkyl group, preferably a $C_{1\text{-}}C_{20}$ fluorine-containing alkyl group, and more preferably a $C_{1\text{-}}C_{12}$ fluorine-containing alkyl group. The fluorine-containing alkyl group may contain one or more fluorine atoms, and preferably two or more fluorine atoms, in its structure. The fluorine-containing alkyl group is preferably one in which at least one hydrogen atom in a primary or secondary alkyl group is replaced with a fluorine atom, and more preferably one in which at least one hydrogen atom in a primary alkyl group is replaced with a fluorine atom. In another embodiment, L is preferably a group represented by the following Formula (I).

Formula (I):

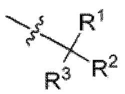 (I)

wherein $R^1$ and $R^2$ are the same or different, and each represents a hydrogen atom or $C_{1\text{-}}C_8$ fluorine-containing alkyl group, and $R^3$ represents a $C_{1\text{-}}C_{20}$ fluorine-containing alkyl group.

In Formula (I), $R^1$ and $R^2$ are the same or different, and each represents a hydrogen atom or a $C_{1\text{-}}C_8$ fluorine-containing alkyl group, and preferably a hydrogen atom or a $C_1$ or $C_2$ fluorine-containing alkyl group. In the configuration of the present invention, the combination with an amidate compound represented by Formula (2), particularly an amidate compound represented by Formula (2a), has excellent low-temperature curing properties. Therefore, $R^1$ and $R^2$ are particularly preferably hydrogen atoms. $R^3$ represents a $C_{1\text{-}}C_{20}$ fluorine-containing alkyl group, preferably a $C_{1\text{-}}C_8$ fluorine-containing alkyl group, more preferably a $C_{1\text{-}}C_4$ fluorine-containing alkyl group, and particularly preferably a $C_1$ fluorine-containing alkyl group. In $R^1$, $R^2$, and $R^3$, the fluorine-containing alkyl group may contain one or more fluorine atoms, and preferably two or more fluorine atoms, in its structure.

Specific examples of the $C_1$-$C_{40}$ fluorine-containing alkyl group include various isomers of difluoroethyl groups, such as monofluoromethyl, difluoromethyl, trifluoromethyl, and 2,2-difluoroethyl; various isomers of trifluoroethyl groups, such as 2,2,2-trifluoroethyl; various isomers of tetrafluoroethyl groups; various isomers of pentafluoroethyl and difluoropropyl groups; various isomers of trifluoropropyl groups; various isomers of hexafluoropropyl groups, such as 1,1,1,3,3,3-hexafluoro-2-propyl; various isomers of heptafluorobutyl, such as 2,2,3,3,4,4,4-heptafluorobutyl; various isomers of octafluoropentyl groups, such as 2,2,3,3,4,4,5,5-octafluoro-1-pentyl; various isomers of nonafluoropentyl groups, such as 2,2,3,3,4,4,5,5,5-nonafluoro-1-pentyl; various isomers of undecafluorohexyl groups, such as 2,2,3,3,4,4,5,5,6,6,6-undecafluoro-1-hexyl; various isomers of dodecafluoroheptyl groups, such as 2,2,3,3,4,4,5,5,6,6,7,7-dodecafluoro-1-heptyl; various isomers of tridecafluoroheptyl groups, such as 2,2,3,3,4,4,5,5,6,6,7,7,7-tridecafluoro-1-heptyl; various isomers of pentadecafluorooctyl groups, such as 2,2,3,3,4,4,5,5,6,6,7,7,8,8,8-pentadecafluoro-1-octyl; and the like. In the present invention, the blocked isocyanate composition of the present invention obtained by combination with the amidate compound (2), particularly the amidate compound represented by Formula (2a), has excellent low-temperature curing properties. Therefore, the $C_1$-$C_{40}$ fluorine-containing alkyl group is preferably a 2,2-difluoroethyl group, a 2,2,2-trifluoroethyl group, a 1,1,1,3,3,3-hexafluoro-2-propyl group, or a 2,2,3,3,4,4,4-heptafluorobutyl group; more preferably a 2,2-difluoroethyl group, a 2,2,2-trifluoroethyl group, or a 2,2,3,3,4,4,4-heptafluorobutyl group; and particularly preferably a 2,2-difluoroethyl group or a 2,2,2-trifluoroethyl group.

The fluoroalcohol-blocked isocyanate compound (BI) is more preferably a blocked isocyanate compound represented by Formula (1a) (hereinafter also referred to as the blocked isocyanate compound (1a)).

Formula (1a):

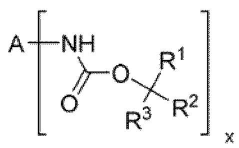

(1a)

wherein A, $R^1$, $R^2$, $R^3$, and x are each as defined above.

Specific examples of the fluoroalcohol-blocked isocyanate compound (BI) include the following.

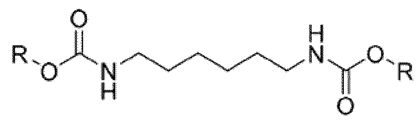

| R | |
|---|---|
| $CF_3CH_2$ | (1-1-1) |
| $CF_2HCH_2$ | (1-1-2) |
| $CF_3CF_2CF_2CH_2$ | (1-1-3) |
| $(CF_3)_2CH$ | (1-1-4) |

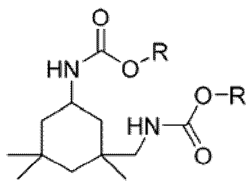

| R | |
|---|---|
| $CF_3CH_2$ | (1-2-1) |

(continued)

| R | |
|---|---|
| $CF_2HCH_2$ | (1-2-2) |
| $CF_3CF_2CF_2CH_2$ | (1-2-3) |
| $(CF_3)_2CH$ | (1-2-4) |

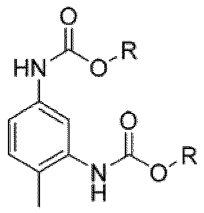

| R | |
|---|---|
| $CF_3CH_2$ | (1-3-1) |
| $CF_2HCH_2$ | (1-3-2) |
| $CF_3CF_2CF_2CH_2$ | (1-3-3) |
| $(CF_3)_2CH$ | (1-3-4) |

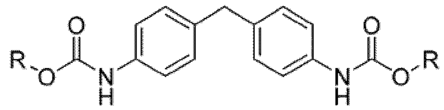

| R | |
|---|---|
| $CF_3CH_2$ | (1-4-1) |
| $CF_2HCH_2$ | (1-4-2) |
| $CF_3CF_2CF_2CH_2$ | (1-4-3) |
| $(CF_3)_2CH$ | (1-4-4) |

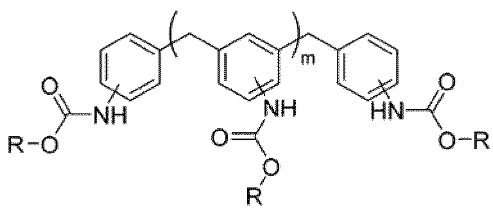

| R | |
|---|---|
| $CF_3CH_2$ | (1-5-1) |
| $CF_2HCH_2$ | (1-5-2) |
| $CF_3CF_2CF_2CH_2$ | (1-5-3) |
| $(CF_3)_2CH$ | (1-5-4) |

In Formulas (1-5-1) to (1-5-4), m is an integer of 0 to 4.

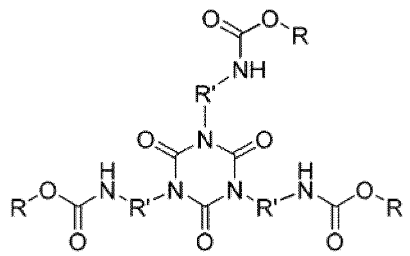

| R’ | R | | R’ | R | |
|---|---|---|---|---|---|
| 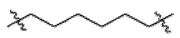 | $CF_3CH_2$ | (1-6-1) | 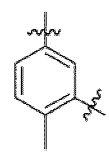 | $CF_3CH_2$ | (1-8-1) |
| | $CF_2HCH_2$ | (1-6-2) | | $CF_2HCH_2$ | (1-8-2) |
| | $CF_3CF_2CF_2CH_2$ | (1-6-3) | | $CF_3CF_2CF_2CH_2$ | (1-8-3) |
| | $(CF_3)_2CH$ | (1-6-4) | | $(CF_3)_2CH$ | (1-8-4) |
| 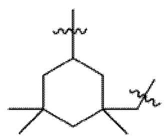 | $CF_3CH_2$ | (1-7-1) | | | |
| | $CF_2HCH_2$ | (1-7-2) | | | |
| | $CF_3CF_2CF_2CH_2$ | (1-7-3) | | | |
| | $(CF_3)_2CH$ | (1-7-4) | | | |

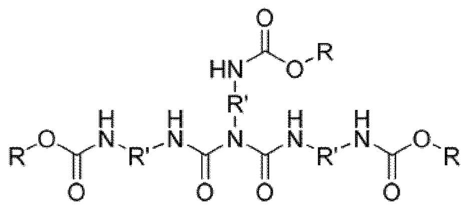

| R’ | R | | R’ | R | |
|---|---|---|---|---|---|
| 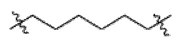 | $CF_3CH_2$ | (1-9-1) | 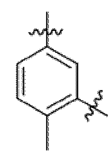 | $CF_3CH_2$ | (1-11-1) |
| | $CF_2HCH_2$ | (1-9-2) | | $CF_2HCH_2$ | (1-11-2) |
| | $CF_3CF_2CF_2CH_2$ | (1-9-3) | | $CF_3CF_2CF_2CH_2$ | (1-11-3) |
| | $(CF_3)_2CH$ | (1-9-4) | | $(CF_3)_2CH$ | (1-11-4) |
| 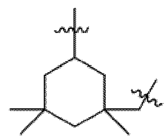 | $CF_3CH_2$ | (1-10-1) | | | |
| | $CF_2HCH_2$ | (1-10-2) | | | |
| | $CF_3CF_2CF_2CH_2$ | (1-10-3) | | | |
| | $(CF_3)_2CH$ | (1-10-4) | | | |

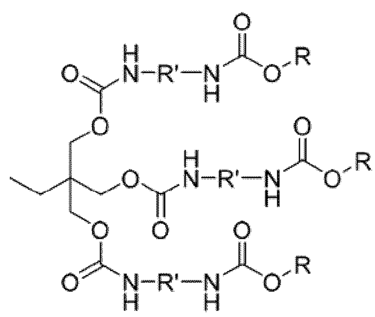

| R' | R | | R' | R | |
|---|---|---|---|---|---|
| 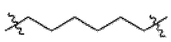 | $CF_3CH_2$ | (1-12-1) | 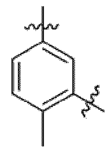 | $CF_3CH_2$ | (1-14-1) |
| | $CF_2HCH_2$ | (1-12-2) | | $CF_2HCH_2$ | (1-14-2) |
| | $CF_3CF_2CF_2CH_2$ | (1-12-3) | | $CF_3CF_2CF_2CH_2$ | (1-14-3) |
| | $(CF3)_2CH$ | (1-12-4) | | $(CF_3)_2CH$ | (1-14-4) |
| 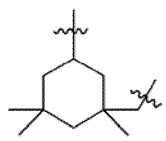 | $CF_3CH_2$ | (1-13-1) | | | |
| | $CF_2HCH_2$ | (1-13-2) | | | |
| | $CF_3CF_2CF_2CH_2$ | (1-13-3) | | | |
| | $(CF_3)_2CH$ | (1-13-4) | | | |

In the present invention, the isocyanate compound is a compound corresponding to a raw material for producing the fluoroalcohol-blocked isocyanate compound (BI), and is a compound having an isocyanate group that is not blocked with a fluoroalcohol compound. The isocyanate compound (hereinafter referred to as the isocyanate compound (P)) may be a compound having at least one isocyanate group in the molecule, preferably a compound having two or more isocyanate groups in the molecule (a polyisocyanate compound), more preferably a compound having 2 or more and 20 or less isocyanate groups in the molecule, and even more preferably an isocyanate compound represented by Formula (3) (hereinafter referred to as the isocyanate compound (3)).

$$A\left[NCO\right]_x \quad (3)$$

In Formula (3), A and x are each as defined above.

Examples of the isocyanate compound (P) include (i) aliphatic polyisocyanates, (ii) alicyclic polyisocyanates, (iii) aromatic polyisocyanates, (iv) aromatic aliphatic polyisocyanates, and (v) modified isocyanates formed from at least one member selected from the group consisting of aliphatic polyisocyanates, alicyclic polyisocyanates, aromatic polyisocyanates, and aromatic aliphatic polyisocyanates.

Examples of aliphatic polyisocyanates include aliphatic diisocyanate, lysine triisocyanate, 4-isocyanatemethyl-1,8-octamethylene diisocyanate, and bis(2-isocyanateethyl) 2-isocyanate glutarate.

Preferable aliphatic diisocyanates are those having 4 to 30 carbon atoms. Examples include 1,4-tetramethylene diisocyanate, 1,6-hexamethylene diisocyanate (hereinafter referred to as HDI), 2,2,4-trimethylhexamethylene diisocyanate, 2,4,4-trimethylhexamethylene diisocyanate, lysine diisocyanate, and the like; HDI is preferred. The aliphatic polyisocyanates may be used singly or in combination of two or more.

Preferable alicyclic polyisocyanates are, for example, those having 8 to 30 carbon atoms. Specific examples include 1,3-bis(isocyanatomethyl)cyclohexane, 1,4-bis(isocyanatomethyl)cyclohexane, isophorone diisocyanate (hereinafter referred to as the IPDI), bis(10-isocyanatocyclohexyl)methane, norbornane diisocyanate, dimer acid diisocyanate, and the like; IPDI is preferred. The alicyclic polyisocyanates may be used singly or in combination of two or more.

Examples of aromatic polyisocyanates include aromatic diisocyanates and polymethylene polyphenyl polyisocyanates (hereinafter referred to as "polymeric MDI"). Examples of aromatic diisocyanates include 2,4'-diphenylmethane diisocyanate, 4,4'-diphenylmethane diisocyanate, crude diphenylmethane diisocyanate, 1,4-phenylene diisocyanate, 2,4-tolylene diisocyanate, 2,6-tolylene diisocyanate, 3,3'-dimethyl-4,4'-diisocyanatobiphenyl, 3,3'-dimethyl-4,4'-diisocyanatodiphenylmethane, 1,5-naphthylene diisocyanate, and the like. The aromatic polyisocyanates may be used singly or in combination of two or more. Preferred in terms of industrial availability are 2,4-tolylene diisocyanate, 2,6-tolylene diisocyanate, 4,4'-diphenylmethane diisocyanate, and polymeric MDI.

Examples of aromatic aliphatic polyisocyanates include 1,3-xylylene diisocyanate, 1,4-xylylene diisocyanate, $\alpha,\alpha,\alpha',\alpha'$-tetramethylxylylene diisocyanate, and the like. The aromatic aliphatic polyisocyanates may be used singly or in combination of two or more.

Examples of modified isocyanates include 2- to 20-mer oligomers of the above isocyanate compounds produced by forming biuret bonds, urea bonds, isocyanurate bonds, uretdione bonds, urethane bonds, allophanate bonds, oxadiazinetrione bonds, etc.; and isocyanates obtained by blocking some isocyanate groups of the above isocyanate compounds or modified isocyanates with known blocking agents.

When a compound having one isocyanate group in the molecule is used as the isocyanate compound (P), it is preferable to use an isocyanate in which other isocyanate groups in the molecule are blocked with known blocking agents.

Modified isocyanate compounds having a biuret bond included in the modified isocyanates are obtained by reacting a so-called biuretizing agent, such as water, tert-butanol, or urea, with an isocyanate compound at a molar ratio of the biuretizing agent/isocyanate groups in the isocyanate compound of about 1/2 to about 1/100, followed by purification by removing the unreacted isocyanate compound.

Examples of modified isocyanate compounds having a biuret bond include a biuret modified product of 1,6-hexamethylene diisocyanate (HDI), a biuret modified product of isophorone diisocyanate (IPDI), and a biuret modified product of toluene diisocyanate (TDI) shown below. Commercial products include Desmodur N75, Desmodur N100, and Desmodur N3200 (all produced by Sumika Covestro Urethane Co., Ltd.); Duranate 24A-100, Duranate 22A-75P, and Duranate 21S-75E (all produced by Asahi Kasei Corporation); and the like.

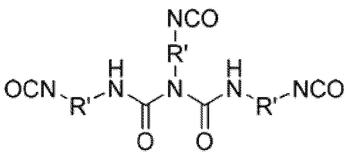 R' = 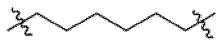 , 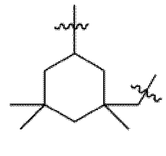 , 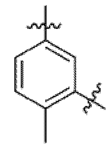

Modified isocyanate compounds having an isocyanurate bond included in the modified isocyanates are obtained, for example, by performing the cyclic trimerization reaction using a catalyst etc., stopping the reaction when the conversion rate reaches about 5 to about 80 mass%, and removing the unreacted polyisocyanate for purification. In this case, a mono- to hexavalent alcohol compound can be used in combination.

The catalyst for the above isocyanuration reaction is generally preferably a basic catalyst. Examples of the catalyst include the following:

(1) hydroxides of tetraalkylammonium, such as tetramethylammonium, tetraethylammonium, and trimethylbenzylammonium; and organic weak acid salts, such as acetic acid and capric acid;
(2) hydroxides of hydroxyalkylammonium, such as trimethylhydroxypropylammonium, trimethylhydroxyethylammonium, triethylhydroxypropylammonium, and triethylhydroxyethylammonium; and organic weak acid salts, such as acetic acid and capric acid;
(3) metal salts of alkyl carboxylic acids with, for example, tin, zinc, and lead;
(4) metal alcoholates of sodium, potassium, etc.;
(5) aminosilyl group-containing compounds, such as hexamethyldisilazane;
(6) Mannich bases;
(7) combination of tertiary amines and epoxy compounds;
(8) phosphorus-based compounds, such as tributylphosphine.

These can be used in combination of two or more.

If the catalyst may adversely affect paints or coating film properties, the catalyst may be neutralized with an acidic compound. Examples of acidic compounds include inorganic acids, such as hydrochloric acid, phosphorous acid, and phosphoric acid; sulfonic acids or derivatives thereof, such as methanesulfonic acid, p-toluenesulfonic acid, p-toluenesulfonic acid methyl ester, and p-toluenesulfonic acid ethyl ester; ethyl phosphate, diethyl phosphate, isopropyl phosphate, diisopropyl phosphate, butyl phosphate, dibutyl phosphate, 2-ethylhexyl phosphate, di(2-ethylhexyl)phosphate, isodecyl phosphate, diisodecyl phosphate, oleyl acid phosphate, tetracosyl acid phosphate, ethyl glycol acid phosphate, butyl pyrophosphate, butyl phosphite, and the like. These may be used in combination of two or more.

Examples of modified isocyanate compounds having an isocyanurate bond include isocyanurate-modified HDI, isocyanurate-modified IPDI, and isocyanurate-modified TDI shown below. Commercial products include Sumidur N3300, Desmodur 3900, Desmodur Z4470BA, Desmodur XP2763, Desmodur IL1351BA, and Desmodur HLBA (all produced by Sumika Covestro Urethane Co., Ltd.); Duranate TPA-100, Duranate MFA-75B, Duranate TUL-100, and Duranate TSA-100 (all produced by Asahi Kasei Corporation); and the like.

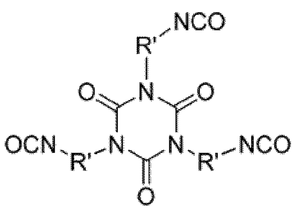 R' = 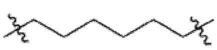 , 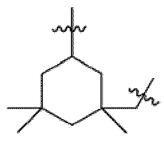 , 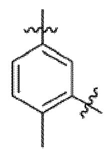

A modified isocyanate compound having a urethane bond included in the modified isocyanates is obtained, for example, by reacting a di- to hexavalent alcohol-based compound, such as trimethylolpropane (hereinafter referred to as TMP), with a polyisocyanate at a molar ratio of hydroxyl groups in the alcohol-based compound/isocyanate groups in the polyisocyanate of about 1/2 to about 1/100, and then removing the unreacted polyisocyanate for purification. Removal of the unreacted polyisocyanate for purification is not necessarily required.

Examples of modified isocyanate compounds having a urethane bond include a reaction product of HDI and TMP, a reaction product of IPDI and TMP, and a reaction product of TDI and TMP shown below. Commercial products include Sumidur N3300, Desmodur 3900, Desmodur Z4470BA, Desmodur XP2763, Desmodur IL1351BA, and Desmodur HLBA (all produced by Sumika Covestro Urethane Co., Ltd.); Duranate TPA-100, Duranate MFA-75B, Duranate TUL-100, and Duranate TSA-100 (all produced by Asahi Kasei Corporation); and the like.

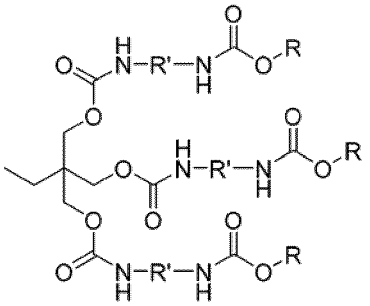

R' = 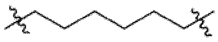 , 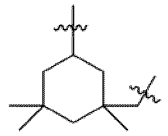 , 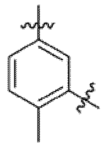

Examples of known blocking agents for isocyanates in which some isocyanate groups of isocyanate compounds or modified isocyanate compounds are blocked with known blocking agents include phenols, such as phenol, thiophenol, methylthiophenol, xylenol, cresol, resorcinol, nitrophenol, and chlorophenol; oximes, such as acetone oxime, methyl ethyl ketone oxime, and cyclohexanone oxime; alcohols, such as methanol, ethanol, n-propyl alcohol, isopropyl alcohol, n-butyl alcohol, isobutyl alcohol, t-butyl alcohol, t-pentanol, ethylene glycol monoethyl ether, ethylene glycol monobutyl ether, diethylene glycol monoethyl ether, propylene glycol monomethyl ether, and benzyl alcohol; pyrazoles, such as 3,5-dimethyl pyrazole and 1,2-pyrazole; triazoles, such as 1,2,4-triazole; halogen-substituted alcohols, such as ethylene chlorohydrin and 1,3-dichloro-2-propanol; lactams, such as ε-caprolactam, δ-valerolactam, γ-butyrolactam, and β-propyllactam; active methylene compounds, such as methyl acetoacetate, ethyl acetoacetate, acetylacetone, methyl malonate, and ethyl malonate; and the like. Other examples include amines, imides, mercaptans, imines, ureas, diaryls, and the like.

The fluoroalcohol compound (hereinafter referred to as the fluoroalcohol compound (F)) that blocks an isocyanate group of the isocyanate compound (P) constituting the fluoroalcohol-blocked isocyanate compound (BI) is explained.

The fluoroalcohol compound (F) is an alcohol compound having one or more fluorine atoms in the molecule. In the present invention, the blocked isocyanate composition of the present invention obtained by combining the amidate compound (2), particularly the amidate compound represented by Formula (2a), with the fluoroalcohol-blocked isocyanate compound (BI) has excellent low-temperature curing properties.

The fluoroalcohol compound (F) is preferably a compound in which one or more hydrogen atoms in a primary or secondary alcohol compound are replaced with fluorine atoms, more preferably a compound in which one or more hydrogen atoms in a primary alcohol compound are replaced with fluorine atoms. In another embodiment of the present invention, the blocked isocyanate composition of the present invention obtained by combination with the amidate compound (2), particularly the amidate compound represented by Formula (2a), has excellent low-temperature curing properties; thus, the fluoroalcohol compound (F) is preferably a fluoroalcohol compound represented by Formula (4) (hereinafter also referred to as the fluoroalcohol compound (4)), and more preferably a fluoroalcohol compound represented by Formula (4a) (hereinafter also referred to as the fluoroalcohol compound (4a)).

Formula (4):

HO-L (4)

wherein L is as defined above.

Formula (4a):

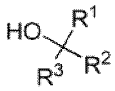 (4a)

wherein $R^1$, $R^2$, and $R^3$ are each as defined above.

Specific examples of the fluoroalcohol compound (F) include various isomers of difluoroethyl alcohols, such as monofluoromethyl alcohol, difluoromethyl alcohol, trifluoromethyl alcohol, and 2,2-difluoroethanol; various isomers of trifluoroethyl alcohols, such as 2,2,2-trifluoroethanol; various isomers of tetrafluoroethyl alcohols; various isomers of pentafluoroethyl alcohols and difluoropropyl alcohols; various isomers of trifluoropropyl alcohols; various isomers of hexafluoropropanols, such as 1,1,1,3,3,3-hexafluoro-2-propanol; various isomers of heptafluorobutanols, such as 2,2,3,3,4,4,4-heptafluorobutanol; various isomers of octafluoropentanols, such as 2,2,3,3,4,4,5,5-octafluoro-1-pentanol; various isomers of nonafluoropentanols, such as 2,2,3,3,4,4,5,5,5-nonafluoro-1-pentanol; various isomers of undecafluorohexanols, such as 2,2,3,3,4,4,5,5,6,6,6-undecafluoro-1-hexanol; various isomers of dodecafluoroheptanols, such as 2,2,3,3,4,4,5,5,6,6,7,7-dodecafluoro-1-heptanol; various isomers of tridecafluoroheptanols, such as 2,2,3,3,4,4,5,5,6,6,7,7,7-tridecafluoro-1-heptanol; various isomers of pentadecafluorooctanols, such as 2,2,3,3,4,4,5,5,6,6,7,7,8,8,8-pentadecafluoro-1-octanol; and the like. In the present invention, the blocked isocyanate composition of the present invention obtained by combination with the amidate compound (2), particularly the amidate compound represented by Formula (2a), has excellent low-temperature curing properties. Thus, preferable are 2,2-difluoroethanol, 2,2,2-trifluoroethanol, 1,1,1,3,3,3-hexafluoro-2-propanol, and 2,2,3,3,4,4,4-heptafluorobutanol; more preferable are 2,2-difluoroethanol, 2,2,2-trifluoroethanol, and 2,2,3,3,4,4,4-heptafluorobutanol; and particularly preferable are 2,2-difluoroethanol and 2,2,2-trifluoroethanol.

The fluoroalcohol-blocked isocyanate compound (BI) may be a commercial product, or may be produced, for example, by the following method.

The fluoroalcohol-blocked isocyanate compound (BI) can be produced by reacting the isocyanate compound (P) and the fluoroalcohol compound (F) optionally in the presence of a urethanization catalyst and a solvent. In a preferred embodiment, the blocked isocyanate compound (1) can be produced by reacting the isocyanate compound represented by Formula (3) and the fluoroalcohol compound represented by Formula (4). In a more preferred embodiment, the blocked isocyanate compound (1a) can be produced by reacting the isocyanate compound represented by Formula (3) and the fluoroalcohol compound represented by Formula (4a) .

The amount of the fluoroalcohol compound (F) used in the production of the fluoroalcohol-blocked isocyanate compound (BI) is generally 0.8 to 10 mol, and preferably 0.8 to 1.2 mol, per mol of isocyanate groups in the isocyanate compound (P). An excessive amount of the fluoroalcohol compound (F) may be used as a reaction solvent.

A urethanization catalyst may or may not be used. When a urethanization catalyst is used, specific examples include tertiary amine catalysts, such as triethylamine, diisopropylethylamine, 1,4-diazabicyclo[2.2.2]octane, 1,8-diazabicyclo[5.4.0]undecene-7, N-methylmorpholine, N-ethylmorpholine, and 1-methylimidazole; carboxylic acid metal salts, such as potassium acetate, potassium octylate, stannous octylate, dibutyl stannic dilaurate, and zinc octylate; and preferably triethylamine.

A solvent may or may not be used. When a solvent is used, specific examples include aromatic hydrocarbons, such as toluene, benzene, and xylene; aliphatic or alicyclic hydrocarbons, such as methylcyclohexane, cyclohexane, hexane, heptane, and octane; halogenated hydrocarbons, such as dichloromethane, chloroform, carbon tetrachloride, and 1,2-dichloroethane; halogenated aromatic hydrocarbons, such as chlorobenzene and dichlorobenzene; ethers, such as diethyl ether, tetrahydrofuran, and 1,4-dioxane; ketones, such as methyl isobutyl ketone; esters, such as butyl acetate; and the like. Preferred among these are aromatic hydrocarbons, ketones, and esters; and particularly preferred are methyl isobutyl ketone and butyl acetate. The solvents can be used as a mixture of two or more, if necessary.

The amount of solvent used is generally 50 parts by mass or less, and preferably 0.1 to 10 parts by mass, per part by mass of the isocyanate compound (P).

The reaction temperature is generally -10°C or higher, preferably 0°C to 150°C, and more preferably 30°C to 100°C. The reaction time is generally 0.1 to 48 hours, and preferably 0.5 to 12 hours.

The reaction may be performed, if necessary, in an inert gas atmosphere, such as nitrogen, argon, or helium, which do not affect the reaction.

After completion of the reaction, the fluoroalcohol-blocked isocyanate compound (BI) can be obtained by removing the solvent and the unreacted fluoroalcohol compound (F) by concentrating or filtering the reaction liquid, and may be purified by recrystallization, column separation, etc., if necessary.

The amidate compound represented by Formula (2) is explained.

In one embodiment, in Formula (2), B is a substituted or unsubstituted hydrocarbon group, preferably a substituted or unsubstituted $C_1$-$C_{100}$ hydrocarbon group, more preferably a substituted or unsubstituted $C_1$-$C_{50}$ hydrocarbon group, and particularly preferably a substituted or unsubstituted $C_1$-$C_{30}$ hydrocarbon group. In another embodiment, B is a substituted or unsubstituted aromatic hydrocarbon group, preferably a substituted or unsubstituted $C_1$-$C_{100}$ aromatic hydrocarbon group, more preferably a substituted or unsubstituted $C_1$-$C_{50}$ aromatic hydrocarbon group, and particularly preferably a substituted or unsubstituted $C_1$-$C_{30}$ aromatic hydrocarbon group.

In the present specification, the "substituted or unsubstituted hydrocarbon groups" include (i) a hydrocarbon group that may have a substituent, (ii) a hydrocarbon group that may be substituted with a heteroatom, and (iii) a hydrocarbon group having a substituent and substituted with a heteroatom. Further, the "substituted or unsubstituted aromatic hydrocarbon groups" include (iv) an aromatic hydrocarbon group that may have a substituent, (v) an aromatic hydrocarbon group that may be substituted with a heteroatom, and (vi) an aromatic hydrocarbon group having a substituent and substituted with a heteroatom.

In B, the unsubstituted hydrocarbon group is, for example, a methyl, ethyl, propyl, isopropyl, butyl, sec-butyl, tert-butyl, pentyl, hexyl, heptyl, decyl, dodecyl, octadecyl, cyclopropyl, cyclopentyl, cyclohexyl, phenyl, naphthyl, benzyl, phenethyl, tolyl, or allyl group.

When B is a substituted hydrocarbon group, examples of substituents include halogen atoms, such as fluorine, chlorine, bromine, and iodine; alkylamino groups, such as methylamino; dialkylamino groups, such as dimethylamino; alkoxy groups, such as methoxy and ethoxy; aryloxy groups, such as phenoxy and naphthyloxy; aralkyloxy groups, such as benzyloxy and naphthylmethoxy; halogenated alkyl groups, such as trifluoromethyl; nitro groups, cyano groups, sulfonyl groups; carbonylamino groups, such as alkylcarbonylamino, alkyloxycarbonylamino, (alkylamino)carbonylamino, and (dialkylamino)carbonylamino; and the like. Moreover, the hydrocarbon group B may be substituted with at least one heteroatom, such as oxygen, nitrogen, or sulfur. When the hydrocarbon group B is substituted with at least one heteroatom, such as oxygen, nitrogen, or sulfur, the hydrocarbon group has at least one group, such as -O-, -N<, -NH-, -S-, or $-SO_2-$, and the hydrocarbon chain is interrupted by such a group.

Examples of the alkyl moiety of the above alkylamino groups, dialkylamino groups, alkoxy groups, halogenated alkyl groups, alkylcarbonylamino groups, alkyloxycarbonylamino groups, (alkylamino)carbonylamino groups, and (dialkylamino)carbonylamino groups include linear or branched $C_1$-$C_{12}$ alkyl groups, such as methyl, ethyl, propyl, isopropyl, n-butyl, isobutyl, sec-butyl, tert-butyl, pentyl, 1-ethylpentyl, heptyl, octyl, and 2-ethylhexyl. The number of carbon atoms in the alkyl group is preferably 1 to 8, and more preferably 1 or 2.

Examples of the aryl moiety of the above aryloxy groups include $C_6$-$C_{10}$ aryl groups. Specific examples of the aryl moiety include a phenyl group, a naphthyl group, and the like.

Examples of the aralkyl moiety of the above aralkyloxy groups include $C_7$-$C_{12}$ aralkyl groups. Specific examples of the aralkyl moiety include a benzyl group, a naphthylmethyl group, and the like.

The number of substituents is 1 to 5, preferably 1 to 3, and more preferably 1 or 2.

G and J are the same or different, and each represents a $C_1$-$C_{80}$ hydrocarbon group that may be substituted with a heteroatom, preferably a $C_1$-$C_{20}$ hydrocarbon group that may be substituted with a heteroatom, and more preferably a $C_1$-$C_{12}$ hydrocarbon group that may be substituted with a heteroatom. In another embodiment, G and J are each preferably a group represented by the following Formula (II), a group represented by the following Formula (III), or an adamantyl group represented by the following Formula (IV).

Formula (II):

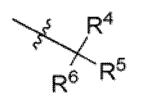

(II)

wherein $R^4$, $R^5$, and $R^6$ are the same or different, and each represents a $C_1$-$C_{20}$ hydrocarbon group that may be substituted with a heteroatom; and $R^4$ and $R^5$, or $R^5$ and $R^6$ may form a ring structure together with the carbon atoms to which they are bonded.

Formula (III):

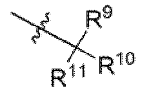

(III)

wherein $R^9$, $R^{10}$, and $R^{11}$ are the same or different, and each represents a $C_1$-$C_{20}$ hydrocarbon group that may be substituted with a heteroatom; and $R^9$ and $R^{10}$, or $R^{10}$ and $R^{11}$ may form a ring structure together with the carbon atoms to which they are bonded.

$R^4$, $R^5$, $R^6$, $R^9$, $R^{10}$, and $R^{11}$ are the same or different, and each represents a $C_1$-$C_{20}$ hydrocarbon group that may be substituted with a heteroatom, preferably a $C_1$-$C_{12}$ hydrocarbon group that may be substituted with a heteroatom, more preferably a $C_1$-$C_8$ hydrocarbon group that may be substituted with a heteroatom, even more preferably a $C_1$-$C_6$ hydrocarbon group that may be substituted with a heteroatom, and particularly preferably a $C_1$-$C_4$ hydrocarbon group that may be substituted with a heteroatom. Examples of hydrocarbon groups include methyl, ethyl, propyl, n-butyl, s-butyl, t-butyl, pentyl, neopentyl, hexyl, octyl, benzyl, and phenyl groups; preferably methyl, ethyl, neopentyl, and phenyl groups; and more preferably methyl and neopentyl groups.

In another embodiment, $R^4$, $R^5$, $R^9$, and $R^{10}$ are preferably the same or different, and each represents a $C_1$-$C_4$ hydrocarbon group that may be substituted with a heteroatom, and $R^6$ and $R^{11}$ are preferably the same or different, and each represents a $C_1$-$C_{12}$ hydrocarbon group that may be substituted with a heteroatom.

The group represented by G or J, or the group represented by $CR^4R^5R^6$ or $CR^9R^{10}R^{11}$- in Formulas (II) and (III) may be an adamantyl group, and may be, for example, a 1-adamantyl group of the following Formula (IV):

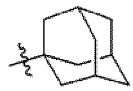

(IV)

$R^7$ and $R^8$ are the same or different, and each represents a hydrogen atom or $C_1$-$C_{20}$ hydrocarbon group that may be substituted with a heteroatom, and preferably a hydrogen atom.

G and $R^7$, or $R^8$ and J may form a ring structure together with the nitrogen and carbon atoms to which they are bonded. $R^7$ and $R^8$ may form a ring structure together with the carbon atoms to which they are bonded. When $R^7$ and $R^8$ form a ring structure together with the carbon atoms to which they are bonded, for example, a benzimidazolium ring structure represented by the following Formula (2x) can be formed.

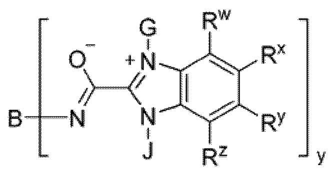

(2x)

wherein B, G, J, and y are as defined above, and $R^w$, $R^x$, $R^y$, and $R^z$ each represent a hydrogen atom or a $C_1$-$C_{20}$ hydrocarbon group.

y is an integer of 1 or more and 20 or less, preferably 1 to 6, more preferably 1 to 4, and particularly preferably 1 or 2.

The amidate compound (2) is preferably an amidate compound represented by the following Formula (2-1), (2-2), or (2-3) .

Formula (2-1):

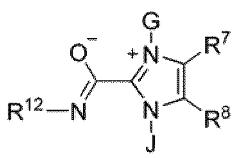

(2-1)

wherein G, J, $R^7$, and $R^8$ are as defined above, and $R^{12}$ represents a substituted or unsubstituted hydrocarbon group.

Formula (2-2):

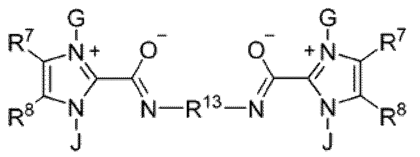

(2-2)

wherein G, J, $R^7$, and $R^8$ are as defined above, and $R^{13}$ represents a substituted or unsubstituted divalent hydrocarbon group.

Formula (2-3):

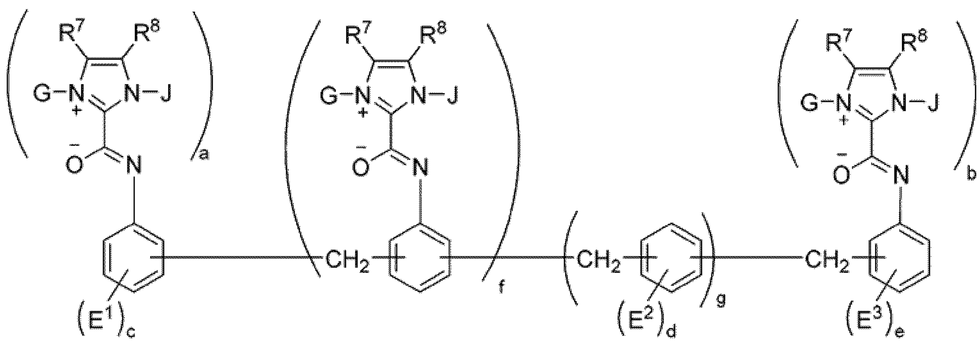

(2-3)

wherein G, J, $R^7$, and $R^8$ are as defined above; $E^1$, $E^2$, and $E^3$ each independently represents a substituted or unsubstituted hydrocarbon group, a halogen atom, an alkylamino group, a dialkylamino group, an alkoxy group, an aryloxy group, an aralkyloxy group, a nitro group, a cyano group, a sulfonyl group, an (alkylamino)carbonylamino group, a (dialkylamino)carbonylamino group, or an isocyanate group; f and g are each independently an integer of 0 to 4; a and b are each 0 or 1; and c, d, and e are each independently an integer of 0 to 4, provided that when f is 0, at least one of a or b is 1.

In Formula (2-1), G, J, $R^7$, and $R^8$ are as defined above.

$R^{12}$ is a substituted or unsubstituted hydrocarbon group, preferably a substituted or unsubstituted $C_1$-$C_{50}$ hydrocarbon group, more preferably a substituted or unsubstituted $C_1$-$C_{30}$ hydrocarbon group, and particularly preferably a substituted or unsubstituted $C_1$-$C_{12}$ hydrocarbon group. Specific examples include methyl, ethyl, propyl, isopropyl, butyl, sec-butyl, tert-butyl, pentyl, hexyl, heptyl, decyl, dodecyl, octadecyl, cyclopropyl, cyclopentyl, cyclohexyl, phenyl, naphthyl, benzyl, phenethyl, tolyl, and allyl groups; and preferably benzyl and phenyl groups.

When $R^{12}$ is substituted, examples of substituents include halogen atoms, such as fluorine, chlorine, bromine, and iodine; alkylamino groups, such as methylamino; dialkylamino groups, such as dimethylamino; alkoxy groups, such as methoxy and ethoxy; aryloxy groups, such as phenoxy and naphthyloxy; aralkyloxy groups, such as benzyloxy and naphthylmethoxy; halogenated alkyl groups, such as trifluoromethyl; nitro groups, cyano groups, sulfonyl groups, alkylcarbonylamino groups, alkyloxycarbonylamino groups, (alkylamino)carbonylamino groups, (dialkylamino)carbonylamino groups, isocyanate groups, and the like. Moreover, the hydrocarbon group $R^{12}$ may be substituted with at least one heteroatom, such as oxygen, nitrogen, or sulfur. When the hydrocarbon group is substituted with at least one heteroatom, such as oxygen, nitrogen, or sulfur, the hydrocarbon group has at least one group, such as -O-, -N<, -NH-, -S-, or - $SO_2$-, and the hydrocarbon chain is interrupted by such a group. When $R^{12}$ is substituted, the number of substituents is preferably 1 to 5, more preferably 1 to 3, and even more preferably 1 to 2.

Examples of the alkyl moiety of the above alkylamino groups, dialkylamino groups, alkoxy groups, halogenated alkyl groups, alkylcarbonylamino groups, alkyloxycarbonylamino groups, (alkylamino)carbonylamino groups, and (dialkylamino)carbonylamino groups include linear or branched $C_1$-$C_{12}$ alkyl groups, such as methyl, ethyl, propyl, isopropyl, butyl, sec-butyl, tert-butyl, pentyl, 1-ethylpentyl, heptyl, octyl, and 2-ethylhexyl. The number of carbon atoms in the alkyl group is preferably 1 to 8, and more preferably 1 or 2.

Examples of the aryl moiety of the above aryloxy groups include $C_6$-$C_{10}$ aryl groups. Specific examples of the aryl moiety include a phenyl group, a naphthyl group, and the like.

Examples of the aralkyl moiety of the above aralkyloxy groups include $C_7$-$C_{12}$ aralkyl groups. Specific examples

of the aralkyl moiety include a benzyl group, a naphthylmethyl group, and the like.

The number of substituents is 1 to 5, preferably 1 to 3, and more preferably 1 or 2.

$R^{12}$ is preferably a hydrocarbon group that may be substituted with a halogen atom or a hydrocarbon group that may be substituted with a heteroatom. Among these, $R^{12}$ is preferably a $C_{1-}C_{50}$ hydrocarbon group that may be substituted with a halogen atom or a $C_{1-}C_{50}$ hydrocarbon group that may be substituted with a heteroatom, more preferably a $C_{1-}C_{30}$ hydrocarbon group that may be substituted with a halogen atom or a $C_{1-}C_{30}$ hydrocarbon group that may be substituted with a heteroatom, and particularly preferably a $C_{1-}C_{12}$ hydrocarbon group that may be substituted with a halogen atom or a $C_{1-}C_{12}$ hydrocarbon group that may be substituted with a heteroatom. The hydrocarbon group is preferably an aromatic hydrocarbon group, such as aryl or aralkyl.

In $R^{12}$, the hydrocarbon group substituted with a halogen atom is, for example, a chlorophenyl group.

In $R^{12}$, the $C_{1-}C_{20}$ hydrocarbon group substituted with a heteroatom is, for example, a 2-methoxymethyl group, a 2-ethoxymethyl group, or a 2-(dimethylamino)methyl group.

In Formula (2-2), G, J, $R^7$, and $R^8$ are as defined above.

$R^{13}$ is a substituted or unsubstituted divalent hydrocarbon group, preferably a substituted or unsubstituted $C_{1-}C_{100}$ divalent hydrocarbon group, more preferably a substituted or unsubstituted divalent $C_{1-}C_{50}$ hydrocarbon group, and particularly preferably a substituted or unsubstituted $C_{1-}C_{30}$ divalent hydrocarbon group. Specific examples include alkylene groups, such as ethylene, n-propylene, n-butylene, n-pentylene, n-hexylene, n-heptylene, n-octylene, n-nonylene, n-decylene, n-dodecylene, n-octadecylene, cyclohexylene, cyclohexane-1,2-diylbismethylene, and cyclohexane-1,4-diylbismethylene; arylene groups, such as m-phenylene, p-phenylene, 2-methyl-m-phenylene, 4-methyl-m-phenylene, and naphthylene; arylalkylene groups, such as phenylethylene, 1-phenylpropylene, 2-phenylpropylene, 1-phenylbutylene, 2-phenylbutylene, naphthylmethylene, and naphthylethylene; arylenealkylene groups, such as p-xylylene, and alkylenearylene groups, such as methylene diphenylene and polymethylene polyphenylene, obtained by suitably combining the above alkylene groups and arylene groups; and the like. These divalent hydrocarbon groups may be repeated or combined to constitute one divalent hydrocarbon group.

When the divalent hydrocarbon group $R^{13}$ is substituted, examples of substituents include halogen atoms, such as fluorine, chlorine, bromine, and iodine; alkylamino groups, such as methylamino; dialkylamino groups, such as dimethylamino; alkoxy groups, such as methoxy and ethoxy; aryloxy groups, such as phenoxy and naphthyloxy; aralkyloxy groups, such as benzyloxy and naphthylmethoxy; halogenated alkyl groups, such as trifluoromethyl; nitro groups, cyano groups, sulfonyl groups, (alkylamino)carbonylamino groups, (dialkylamino)carbonylamino groups, isocyanate groups, and the like. Moreover, the hydrocarbon group $R^{13}$ may be substituted with at least one heteroatom, such as oxygen, nitrogen, or sulfur. When the hydrocarbon group is substituted with at least one heteroatom, such as oxygen, nitrogen, or sulfur, the hydrocarbon group has at least one group, such as -O-, -N<, -NH-, -S-, or $-SO_2-$, and the hydrocarbon chain is interrupted by such a group. When $R^{13}$ is substituted, the number of substituents is preferably 1 to 5, more preferably 1 to 3, and even more preferably 1 to 2.

$R^{13}$ is preferably a divalent hydrocarbon group that may be substituted with a halogen atom or a divalent hydrocarbon group that may be substituted with a heteroatom. Among these, $R^{13}$ is preferably a $C_{1-}C_{100}$ divalent hydrocarbon group that may be substituted with a halogen atom or a $C_{1-}C_{100}$ divalent hydrocarbon group that may be substituted with a heteroatom, more preferably a $C_{1-}C_{50}$ divalent hydrocarbon group that may be substituted with a halogen atom or a $C_{1-}C_{50}$ divalent hydrocarbon group that may be substituted with a heteroatom, and particularly preferably a $C_{1-}C_{30}$ divalent hydrocarbon group that may be substituted with a halogen atom or a $C_{1-}C_{30}$ divalent hydrocarbon group that may be substituted with a heteroatom.

In Formula (2-3), G, J, $R^7$, and $R^8$ are as defined above. $E^1$, $E^2$, and $E^3$ are each independently a substituted or unsubstituted hydrocarbon group, a halogen atom, an alkylamino group, a dialkylamino group, an alkoxy group, an aryloxy group, an aralkyloxy group, a nitro group, a cyano group, a sulfonyl group, an (alkylamino)carbonylamino group, a (dialkylamino)carbonylamino group, or an isocyanate group; preferably a hydrocarbon group that may be substituted with a halogen atom, a hydrocarbon group that may be substituted with a heteroatom, an (alkylamino)carbonylamino group, a (dialkylamino)carbonylamino group, or an isocyanate group; and more preferably an (alkylamino)carbonylamino group or a (dialkylamino)carbonylamino group.

When $E^1$, $E^2$, and $E^3$ are substituted or unsubstituted hydrocarbon groups, they are preferably substituted or unsubstituted $C_{1-}C_{50}$ hydrocarbon groups, more preferably substituted or unsubstituted $C_{1-}C_{30}$ hydrocarbon groups, and even more preferably substituted or unsubstituted $C_{1-}C_{12}$ hydrocarbon groups.

When $E^1$, $E^2$, or $E^3$ is a substituted hydrocarbon group, examples of substituents include halogen atoms, such as fluorine, chlorine, bromine, and iodine; alkylamino groups, such as methylamino; dialkylamino groups, such as dimethylamino; alkoxy groups, such as methoxy and ethoxy; aryloxy groups, such as phenoxy and naphthyloxy; aralkyloxy groups, such as benzyloxy and naphthylmethoxy; halogenated alkyl groups, such as trifluoromethyl; nitro groups, cyano groups, sulfonyl groups; carbonylamino groups, such as alkylcarbonylamino, alkyloxycarbonylamino, (alkylamino)carbonylamino, and (dialkylamino)carbonylamino; and the like.

The hydrocarbon group $E^1$, $E^2$, or $E^3$ may be substituted with at least one heteroatom, such as oxygen, nitrogen,

or sulfur. When the hydrocarbon group $E^1$, $E^2$, or $E^3$ is substituted with at least one heteroatom, such as oxygen, nitrogen, or sulfur, the hydrocarbon group has at least one group, such as - O-, -N<, -NH-, -S-, or $-SO_2-$, and the hydrocarbon chain is interrupted by such a group.

Examples of the alkyl moiety of the above alkylamino groups, dialkylamino groups, alkoxy groups, halogenated alkyl groups, alkylcarbonylamino groups, alkyloxycarbonylamino groups, (alkylamino)carbonylamino groups, and (dialkylamino)carbonylamino groups include linear or branched $C_{1-}C_{12}$ alkyl groups, such as methyl, ethyl, propyl, isopropyl, n-butyl, isobutyl, sec-butyl, tert-butyl, pentyl, 1-ethylpentyl, heptyl, octyl, and 2-ethylhexyl. The number of carbon atoms in the alkyl group is preferably 1 to 8, and more preferably 1 or 2.

Examples of the aryl moiety of the above aryloxy groups include $C_{6-}C_{10}$ aryl groups. Specific examples of the aryl moiety include a phenyl group, a naphthyl group, and the like.

Examples of the aralkyl moiety of the above aralkyloxy groups include $C_{7-}C_{12}$ aralkyl groups. Specific examples of the aralkyl moiety include a benzyl group, a naphthylmethyl group, and the like.

The number of substituents is 1 to 5, preferably 1 to 3, and more preferably 1 or 2.

When $E^1$, $E^2$, or $E^3$ is a hydrocarbon group that may be substituted with a halogen atom or a hydrocarbon group that may be substituted with a heteroatom, it is preferably a $C_{1-}C_{50}$ hydrocarbon group that may be substituted with a halogen atom or a $C_{1-}C_{50}$ hydrocarbon group that may be substituted with a heteroatom, more preferably a $C_{1-}C_{30}$ hydrocarbon group that may be substituted with a halogen atom or a $C_{1-}C_{30}$ hydrocarbon group that may be substituted with a heteroatom, and particularly preferably a $C_{1-}C_{12}$ hydrocarbon group that may be substituted with a halogen atom or a $C_{1-}C_{12}$ hydrocarbon group that may be substituted with a heteroatom.

Moreover, the hydrocarbon group in the hydrocarbon group that may be substituted with a halogen atom or the hydrocarbon group that may be substituted with a heteroatom is preferably an aromatic hydrocarbon group, such as aryl or arylalkyl. Specific examples of the hydrocarbon group in the hydrocarbon group that may be substituted with a halogen atom or the hydrocarbon group that may be substituted with a heteroatom include methyl, ethyl, propyl, isopropyl, butyl, sec-butyl, tert-butyl, pentyl, hexyl, heptyl, decyl, dodecyl, octadecyl, cyclopropyl, cyclopentyl, cyclohexyl, phenyl, naphthyl, benzyl, phenethyl, tolyl, and allyl groups; and preferably benzyl and phenyl groups.

When $E^1$, $E^2$, or $E^3$ is a hydrocarbon group substituted with a halogen atom, examples of the halogen atom include fluorine, chlorine, bromine, iodine, and the like. Further, the hydrocarbon group $E^1$, $E^2$, or $E^3$ may be substituted with a heteroatom, such as oxygen, nitrogen, or sulfur. When the hydrocarbon group $E^1$, $E^2$, or $E^3$ is substituted with at least one heteroatom, such as oxygen, nitrogen, or sulfur, the hydrocarbon group has at least one group, such as -O-, -N<, -NH-, -S-, or - $SO_2$-, and the hydrocarbon chain is interrupted by such a group.

In $E^1$, $E^2$, or $E^3$, the hydrocarbon group substituted with a halogen atom is, for example, a chlorophenyl group.

In $E^1$, $E^2$, or $E^3$, the $C_{1-}C_{20}$ hydrocarbon group substituted with a heteroatom is, for example, a 2-methoxymethyl group, a 2-ethoxymethyl group, or a 2-(dimethylamino)methyl group.

When $E^1$, $E^2$, and $E^3$ are each independently a halogen atom, examples of the halogen atom include fluorine, chlorine, bromine, iodine, and the like.

When $E^1$, $E^2$, and $E^3$ are alkylamino groups, dialkylamino groups, alkoxy groups, (alkylamino)carbonylamino groups, or (dialkylamino)carbonylamino groups, examples of the alkyl moiety of these groups include linear or branched $C_{1-}C_6$ alkyl groups, such as methyl, ethyl, n-propyl, isopropyl, n-butyl, isobutyl, sec-butyl, tert-butyl, and n-pentyl. The number of carbon atoms in the alkyl group is preferably 1 to 3, and more preferably 1 or 2.

When $E^1$, $E^2$, and $E^3$ are aryloxy groups, examples of the aryl moiety include $C_{6-}C_{10}$ aryl groups. Specific examples of the aryl moiety include a phenyl group, a naphthyl group, and the like.

When $E^1$, $E^2$, and $E^3$ are aralkyloxy groups, examples of the aralkyl moiety of the above aralkyloxy groups include $C_{7-}C_{12}$ aralkyl groups. Specific examples of the aralkyl moiety include a benzyl group, a naphthylmethyl group, and the like.

f and g each independently represent an integer of 0 to 4. a and b are each 0 or 1, and c, d, and e each independently represent an integer of 0 to 4. However, when f is 0, at least one of a or b is 1.

Moreover, the amidate compound represented by Formula (2) is preferably an amidate compound represented by Formula (2a).

Formula (2a):

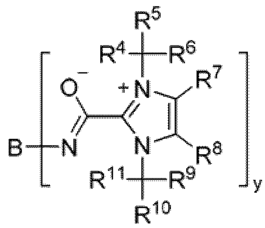

(2a)

wherein B, $R^4$, $R^5$, $R^6$, $R^7$, $R^8$, $R^9$, $R^{10}$, $R^{11}$, and y are as defined above; $R^4$ and $R^5$, $R^5$ and $R^6$, $R^6$ and $R^7$, $R^7$ and $R^8$, $R^8$ and $R^9$, $R^9$ and $R^{10}$, or $R^{10}$ and $R^{11}$ may form a ring structure together with the carbon atoms to which they are bonded; and the group represented by $CR^4R^5R^6$ or the group represented by $CR^9R^{10}R^{11}$ may be an adamantyl group.

When $R^4$ and $R^5$, $R^5$ and $R^6$, $R^7$ and $R^8$, $R^9$ and $R^{10}$, or $R^{10}$ and $R^{11}$ form a ring structure together with the carbon atoms to which they are bonded, for example, a benzimidazolium ring structure shown below can be formed.

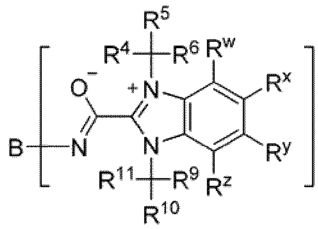

(2ax)

wherein B, $R^4$, $R^5$, $R^6$, $R^9$, $R^{10}$, $R^{11}$, and y are as defined above, and $R^w$, $R^x$, $R^y$, and $R^z$ each represent a hydrogen atom or a $C_1$-$C_{20}$ hydrocarbon group.

Further, the group represented by $CR^4R^5R^6$ or the group represented by $CR^9R^{10}R^{11}$- may be an adamantyl group. In this case, for example, a structure having a 1-adamantyl group represented by the following Formula (2ay) can be formed.

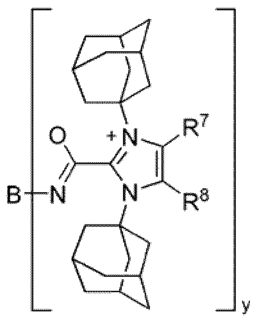

(2ay)

wherein B, $R^7$, $R^8$, and y are as defined above.

The amidate compound represented by Formula (2a) is preferably an amidate compound represented by Formula (2a-1), (2a-2), or (2a-3).

Formula (2a-1):

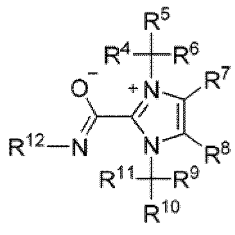

(2a-1)

wherein $R^4$, $R^5$, $R^6$, $R^7$, $R^8$, $R^9$, $R^{10}$, $R^{11}$, and $R^{12}$ are as defined above, and the group represented by $CR^4R^5R^6$ or the group represented by $CR^9R^{10}R^{11}$ may be an adamantyl group.

Formula (2a-2):

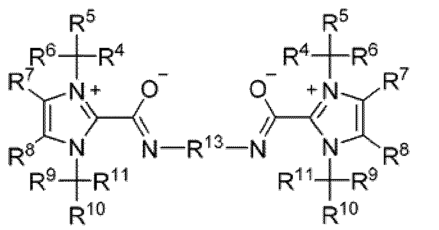

(2a-2)

wherein $R^4$, $R^5$, $R^6$, $R^7$, $R^8$, $R^9$, $R^{10}$, $R^{11}$, and $R^{13}$ are as defined above, and the group represented by $CR^4R^5R^6$ or the group represented by $CR^9R^{10}R^{11}$ may be an adamantyl group.

Formula (2a-3):

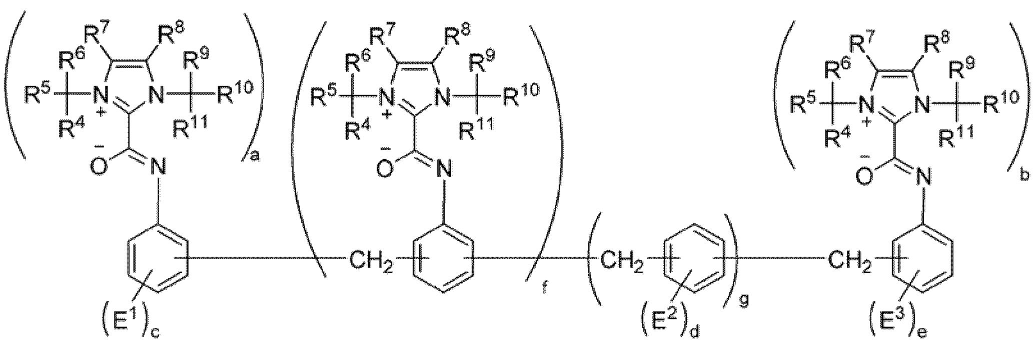

(2a-3)

wherein $R^4$, $R^5$, $R^6$, $R^7$, $R^8$, $R^9$, $R^{10}$, $R^{11}$, $E^1$, $E^2$, $E^3$, a, b, c, d, e, f, and g are as defined above, and the group represented by $CR^4R^5R^6$ or the group represented by $CR^9R^{10}R^{11}$ may be an adamantyl group.

Examples of the amidate compound (2) include the following compounds. In the following specific examples, Me represents a methyl group, iPr represents an isopropyl group, Bu represents an n-butyl group, tBu represents a tert-butyl group, cHex represents a cyclohexyl group, 2EtHex represents a 2-ethylhexyl group, nOct represents an n-octyl group, tOct represents a 1,1,3,3-tetramethylbutyl group, 1Ad represents a 1-adamantyl group, Mes represents a 2,4,6-trimethylphenyl group, Dip represents a 2,6-diisopropylphenyl group, Hept represents an n-heptyl group, 1EtPent represents a 1-ethylpentyl group, and MeO represents a methoxy group.

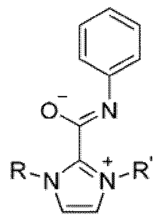

| R | R' | |
|---|---|---|
| Me | Me | (2-1-1) |
| Me | n0ct | (2-1-2) |
| iPr | iPr | (2-1-3) |
| Bu | Bu | (2-1-4) |
| tBu | tBu | (2-1-5) |
| cHex | cHex | (2-1-6) |
| 2EtHex | 2EtHex | (2-1-7) |
| nOct | nOct | (2-1-8) |
| tOct | tOct | (2-1-9) |
| 1Ad | 1Ad | (2-1-10) |
| Mes | Mes | (2-1-11) |
| Dip | Dip | (2-1-12) |

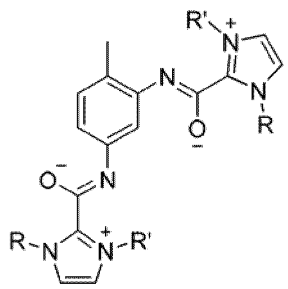

| R | R' | |
|---|---|---|
| Me | Me | (2-2-1) |
| Me | nOct | (2-2-2) |
| iPr | iPr | (2-2-3) |
| Bu | Bu | (2-2-4) |
| tBu | tBu | (2-2-5) |
| cHex | cHex | (2-2-6) |
| 2EtHex | 2EtHex | (2-2-7) |
| nOct | nOct | (2-2-8) |
| tOct | tOct | (2-2-9) |
| 1Ad | 1Ad | (2-2-10) |
| Mes | Mes | (2-2-11) |
| Dip | Dip | (2-2-12) |

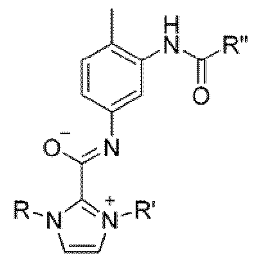

| R | R' | R" | | R | R' | R" | |
|---|---|---|---|---|---|---|---|
| Me | Me | Me | (2-3-1) | 2EtHex | 2EtHex | Me | (2-3-19) |
| Me | Me | Hept | (2-3-2) | 2EtHex | 2EtHex | Hept | (2-3-20) |
| Me | Me | 1Et Pent | (2-3-3) | 2EtHex | 2EtHex | 1EtPent | (2-3-21) |
| Me | n0ct | Me | (2-3-4) | n0ct | n0ct | Me | (2-3-22) |
| Me | n0ct | Hept | (2-3-5) | n0ct | n0ct | Hept | (2-3-23) |
| Me | n0ct | 1EtPent | (2-3-6) | n0ct | n0ct | 1EtPent | (2-3-24) |
| iPr | iPr | Me | (2-3-7) | t0ct | t0ct | Me | (2-3-25) |
| iPr | iPr | Hept | (2-3-8) | tart | t0ct | Hept | (2-3-26) |
| iPr | iPr | 1Et Pent | (2-3-9) | t0ct | t0ct | 1Et Pent | (2-3-27) |
| Bu | Bu | Me | (2-3-10) | 1Ad | 1Ad | Me | (2-3-28) |
| Bu | Bu | Hept | (2-3-11) | 1Ad | 1Ad | Hept | (2-3-29) |
| Bu | Bu | 1EtPent | (2-3-12) | 1Ad | 1Ad | 1Et Pent | (2-3-30) |
| tBu | tBu | Me | (2-3-13) | Mes | Mes | Me | (2-3-31) |
| tBu | tBu | Hept | (2-3-14) | Mes | Mes | Hept | (2-3-32) |
| tBu | tBu | 1EtPent | (2-3-15) | Mes | Mes | 1EtPent | (2-3-33) |
| cHex | cHex | Me | (2-3-16) | Dip | Dip | Me | (2-3-34) |
| cHex | cHex | Hept | (2-3-17) | Dip | Dip | Hept | (2-3-35) |
| cHex | cHex | 1EtPent | (2-3-18) | Dip | Dip | 1EtPent | (2-3-36) |

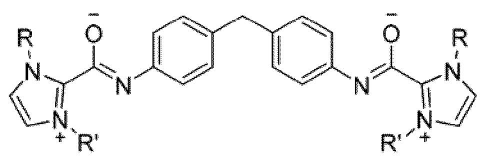

| R | R' | |
|---|---|---|
| Me | Me | (2-4-1) |
| Me | n0ct | (2-4-2) |
| iPr | iPr | (2-4-3) |
| Bu | Bu | (2-4-4) |
| tBu | tBu | (2-4-5) |
| cHex | cHex | (2-4-6) |
| 2EtHex | 2EtHex | (2-4-7) |
| n0ct | n0ct | (2-4-8) |
| t0ct | t0ct | (2-4-9) |
| 1Ad | 1Ad | (2-4-10) |
| Mes | Mes | (2-4-11) |
| Dip | Dip | (2-4-12) |

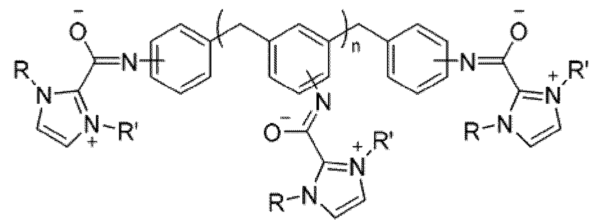

| R | R’ | |
|---|---|---|
| Me | Me | (2-5-1) |
| Me | n0ct | (2-5-2) |
| iPr | iPr | (2-5-3) |
| Bu | Bu | (2-5-4) |
| tBu | tBu | (2-5-5) |
| cHex | cHex | (2-5-6) |
| 2EtHex | 2EtHex | (2-5-7) |
| n0ct | n0ct | (2-5-8) |
| t0ct | t0ct | (2-5-9) |
| 1Ad | 1Ad | (2-5-10) |
| Mes | Mes | (2-5-11) |
| Dip | Dip | (2-5-12) |

In Formulas (2-5-1) to (2-5-12), n is an integer of 0 to 4.

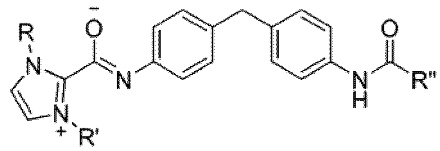

| R | R’ | R" | | R | R’ | R" | |
|---|---|---|---|---|---|---|---|
| Me | Me | Me | (2-6-1) | 2EtHex | 2EtHex | Me | (2-6-19) |
| Me | Me | Hept | (2-6-2) | 2EtHex | 2EtHex | Hept | (2-6-20) |
| Me | Me | lEtPent | (2-6-3) | 2EtHex | 2EtHex | lEt Pent | (2-6-21) |
| Me | n0ct | Me | (2-6-4) | n0ct | n0ct | Me | (2-6-22) |
| Me | n0ct | Hept | (2-6-5) | n0ct | n0ct | Hept | (2-6-23) |
| Me | n0ct | 1EtPent | (2-6-6) | n0ct | n0ct | 1EtPent | (2-6-24) |
| iPr | iPr | Me | (2-6-7) | t0ct | t0ct | Me | (2-6-25) |
| iPr | iPr | Hept | (2-6-8) | t0ct | t0ct | Hept | (2-6-26) |
| iPr | iPr | 1EtPent | (2-6-9) | t0ct | t0ct | 1EtPent | (2-6-27) |
| Bu | Bu | Me | (2-6-10) | 1Ad | lAd | Me | (2-6-28) |
| Bu | Bu | Hept | (2-6-11) | 1Ad | lAd | Hept | (2-6-29) |
| Bu | Bu | lEtPent | (2-6-12) | 1Ad | lAd | 1EtPent | (2-6-30) |
| tBu | tBu | Me | (2-6-13) | Mes | Mes | Me | (2-6-31) |
| tBu | tBu | Hept | (2-6-14) | Mes | Mes | Hept | (2-6-32) |
| tBu | tBu | 1EtPent | (2-6-15) | Mes | Mes | 1EtPent | (2-6-33) |
| cHex | cHex | Me | (2-6-16) | Dip | Dip | Me | (2-6-34) |
| cHex | cHex | Hept | (2-6-17) | Dip | Dip | Hept | (2-6-35) |
| cHex | cHex | lEtPent | (2-6-18) | Dip | Dip | 1EtPent | (2-6-36) |

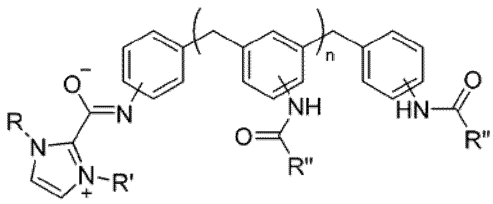

| R | R' | R" | | R | R' | R" | |
|---|---|---|---|---|---|---|---|
| Me | Me | Me | (2-7-1) | 2EtHex | 2EtHex | Me | (2-7-19) |
| Me | Me | Hept | (2-7-2) | 2EtHex | 2EtHex | Hept | (2-7-20) |
| Me | Me | lEt Pent | (2-7-3) | 2EtHex | 2EtHex | 1EtPent | (2-7-21) |
| Me | n0ct | $CH_3$ | (2-7-4) | n0ct | n0ct | Me | (2-7-22) |
| Me | n0ct | Hept | (2-7-5) | n0ct | n0ct | Hept | (2-7-23) |
| Me | n0ct | 1EtPent | (2-7-6) | n0ct | n0ct | 1EtPent | (2-7-24) |
| iPr | iPr | Me | (2-7-7) | t0ct | t0ct | Me | (2-7-25) |
| iPr | iPr | Hept | (2-7-8) | t0ct | t0ct | Hept | (2-7-26) |
| iPr | iPr | 1EtPent | (2-7-9) | t0ct | t0ct | 1EtPent | (2-7-27) |
| Bu | Bu | Me | (2-7-10) | 1Ad | 1Ad | Me | (2-7-28) |
| Bu | Bu | Hept | (2-7-11) | 1Ad | 1Ad | Hept | (2-7-29) |
| Bu | Bu | lEt Pent | (2-7-12) | 1Ad | lAd | 1EtPent | (2-7-30) |
| tBu | tBu | Me | (2-7-1 3) | Mes | Mes | Me | (2-7-31) |
| tBu | tBu | Hept | (2-7-14) | Mes | Mes | Hept | (2-7-32) |
| tBu | tBu | 1EtPent | (2-7-15) | Mes | Mes | 1EtPent | (2-7-33) |
| cHex | cHex | Me | (2-7-16) | Dip | Dip | Me | (2-7-34) |
| cHex | cHex | Hept | (2-7-17) | Dip | Dip | Hept | (2-7-35) |
| cHex | cHex | 1EtPent | (2-7-18) | Dip | Dip | 1EtPent | (2-7-36) |

In Formulas (2-7-1) to (2-5-36), n is an integer of 0 to 4.

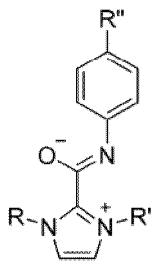

| R | R' | R" | | R | R' | R" | |
|---|---|---|---|---|---|---|---|
| Me | Me | Me | (2-8-1) | 2EtHex | 2EtHex | Me | (2-8-19) |
| Me | Me | Me0 | (2-8-2) | 2EtHex | 2EtHex | Me0 | (2-8-20) |
| Me | Me | Cl | (2-8-3) | 2EtHex | 2EtHex | Cl | (2-8-21) |
| Me | n0ct | Me | (2-8-4) | n0ct | n0ct | Me | (2-8-22) |
| Me | n0ct | Me0 | (2-8-5) | n0ct | n0ct | Me0 | (2 8 23) |
| Me | n0ct | Cl | (2-8-6) | n0ct | n0ct | Cl | (2-8-24) |
| iPr | iPr | Me | (2-8-7) | t0ct | t0ct | Me | (2-8-25) |
| iPr | iPr | Me0 | (2-8-8) | t0ct | t0ct | Me0 | (2-8-26) |
| iPr | iPr | Cl | (2-8-9) | t0ct | t0ct | Cl | (2--8--27) |
| Bu | Bu | Me | (2-8-10) | 1Ad | 1Ad | Me | (2-8-28) |

(continued)

| R | R' | R" | | R | R' | R" | |
|---|---|---|---|---|---|---|---|
| Bu | Bu | Me0 | (2-8-11) | 1Ad | 1Ad | Me0 | (2-8-29) |
| Bu | Bu | Cl | (2-8-12) | 1Ad | 1Ad | Cl | (2-8-30) |
| tBu | tBu | Me | (2-8-13) | Mes | Mes | Me | (2-8-31) |
| tBu | tBu | Me0 | (2-8-14) | Mes | Mes | Me0 | (2-8-32) |
| tBu | tBu | Cl | (2-8-15) | Mes | Mes | Cl | (2-8-33) |
| cHex | cHex | Me | (2-8-16) | Dip | Dip | Me | (2-8-34) |
| cHex | cHex | Me0 | (2-9-17) | Dip | Dip | Me0 | (2-8-35) |
| cHex | cHex | Cl | (2-8-18) | Dip | Dip | Cl | (2-8-36) |

The amidate compound (2) is preferably a compound represented by Formula (2-1-5), (2-1-7), (2-1-8), (2-1-9), (2-1-10), (2-2-5), (2-2-7), (2-2-8), (2-2-9), (2-2-10), (2-3-13), (2-3-15), (2-3-19), (2-3-21), (2-3-22), (2-3-24), (2-3-25), (2-3-27), (2-3-28), (2-3-30), (2-4-5), (2-4-7), (2-4-8), (2-4-9), (2-4-10), (2-5-5), (2-5-7), (2-5-8), (2-5-9), (2-5-10), (2-6-13), (2-6-15), (2-6-19), (2-6-21), (2-6-22), (2-6-24), (2-6-25), (2-6-27), (2-6-28), (2-6-30), (2-8-14), (2-8-20), (2-8-23), (2-8-26), or (2-8-29); more preferably a compound represented by Formula (2-1-5), (2-1-9), (2-1-10), (2-2-5), (2-2-9), (2-2-10), (2-3-13), (2-3-15), (2-3-25), (2-3-27), (2-3-28), (2-3-30), (2-4-5), (2-4-9), (2-4-10), (2-5-5), (2-5-9), (2-5-10), (2-6-13), (2-6-15), (2-6-25), (2-6-27), (2-6-28), (2-6-30), (2-8-14), (2-8-26), or (2-8-29); and particularly preferably a compound represented by Formula (2-1-5), (2-1-9), (2-1-10), or (2-4-9).

The amidate compound (2) can be produced, for example, by the methods shown below.

Method 1: An imidazolium carboxylate represented by Formula (5) is reacted with an ester carbonate represented by Formula (6) (Reaction 1), and the obtained reaction product is reacted with an isocyanate compound represented by Formula (10) (Reaction 2).

Method 2: An imidazolium carboxylate is reacted with an isocyanate compound.

Method 3: The imidazolium-2-carboxylate compound described in PTL 1 is reacted with an isocyanate compound.

Method 4: The imidazolium-2-ylidene compound described in Structural Chemistry, 2013, vol. 24, pp. 2059-2068, is reacted with an isocyanate compound.

Method 1 is explained below.

Reaction 1:

An imidazolium carboxylate represented by the following Formula (5) (hereinafter referred to as the imidazolium carboxylate (5)) is reacted with an ester carbonate represented by the following Formula (6).

Reaction 2:

Then, the obtained reaction product is reacted with an isocyanate compound represented by the following Formula (10) optionally in the presence of a solvent to produce the amidate compound (2).

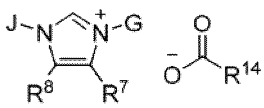 (5)

wherein G, J, $R^7$, and $R^8$ are each as defined above, and $R^{14}$ represents a hydrogen atom or a $C_1$-$C_{20}$ hydrocarbon group that may be substituted with a heteroatom.

Formula (6):

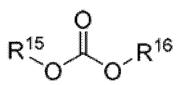 (6)

wherein $R^{15}$ and $R^{16}$ are the same or different, and each represents a $C_1$-$C_6$ hydrocarbon group, and $R^{15}$ and $R^{16}$ may

form a ring structure together with the oxygen atoms to which they are bonded.

Formula (10):

$$B\left[NCO\right]_y \quad (10)$$

wherein B and y are each as defined above.

The imidazolium carboxylate (5) is explained.

In Formula (5), G, J, $R^7$, and $R^8$ are each as defined above. When $R^7$ and $R^8$ form a ring structure together with the carbon atoms to which they are bonded, for example, a benzimidazolium ring structure shown below can be formed.

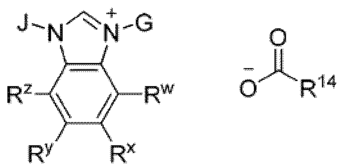 (5x)

wherein G, J, $R^{14}$, $R^w$, $R^x$, $R^y$, and $R^z$ are as defined above.

$R^{14}$ represents a hydrogen atom or a $C_1$-$C_{20}$ hydrocarbon group that may be substituted with a heteroatom, and preferably a $C_1$-$C_{20}$ hydrocarbon group that may be substituted with a heteroatom. The $C_1$-$C_{20}$ hydrocarbon group that may be substituted with a heteroatom is preferably a $C_1$-$C_8$ hydrocarbon group that may be substituted with a heteroatom, and particularly preferably a $C_1$ or $C_2$ hydrocarbon group that may be substituted with a heteroatom. Examples of the hydrocarbon group in the $C_1$-$C_{20}$ hydrocarbon group that may be substituted with a heteroatom include methyl, ethyl, propyl, isopropyl, butyl, sec-butyl, tert-butyl, pentyl, hexyl, heptyl, octyl, 1-ethylpentyl, nonyl, 2-ethylhexyl, undecyl, tridecyl, pentadecyl, heptadecyl, vinyl, allyl, benzyl, cyclohexyl, adamantyl, and phenyl groups; preferably methyl, ethyl, propyl, isopropyl, butyl, heptyl, cyclohexyl, 1-ethylpentyl, and phenyl groups; and particularly preferably methyl, ethyl, heptyl, and 1-ethylpentyl groups.

In $R^{14}$, the heteroatom is, for example, a nitrogen atom, an oxygen atom, a sulfur atom, or the like. When the hydrocarbon group $R^{14}$ is substituted with at least one heteroatom, such as oxygen, nitrogen, or sulfur, the hydrocarbon group has at least one group, such as -O-, -N<, -NH-, -S-, or $-SO_2-$, and the hydrocarbon chain is interrupted by such a group. When the hydrocarbon group $R^{14}$ is substituted with at least one heteroatom, such as oxygen, nitrogen, or sulfur, it is preferable that the hydrocarbon group is substituted with an oxygen atom, and that the hydrocarbon chain is interrupted by an -O- group. In another embodiment, when the hydrocarbon group is substituted with at least one heteroatom, such as oxygen, nitrogen, or sulfur, a hydrocarbon group having a group such as -OH or $-NH_2$ may be formed.

In $R^{14}$, the $C_1$-$C_{20}$ hydrocarbon group substituted with a heteroatom is, for example, a 2-methoxymethyl group, a 2-ethoxymethyl group, or a 2-(dimethylamino)methyl group.

The imidazolium carboxylate (5) is preferably an imidazolium carboxylate represented by the following Formula (5a) :

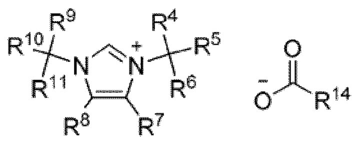 (5a)

wherein $R^4$, $R^5$, $R^6$, $R^7$, $R^8$, $R^9$, $R^{10}$, $R^{11}$, and $R^{14}$ are each as defined above, and $R^4$ and $R^5$, $R^5$ and $R^6$, $R^7$ and $R^8$, $R^9$ and $R^{10}$, or $R^{10}$ and $R^{11}$ may form a ring structure together with the carbon atoms to which they are bonded.

When $R^4$ and $R^5$, $R^5$ and $R^6$, $R^7$ and $R^8$, $R^9$ and $R^{10}$, or $R^{10}$ and $R^{11}$ form a ring structure together with the carbon atoms to which they are bonded, for example, a benzimidazolium ring structure shown below can be formed.

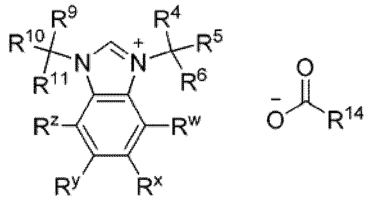 (5ax)

wherein $R^4$, $R^5$, $R^6$, $R^9$, $R^{10}$, $R^{11}$, $R^{14}$, $R^w$, $R^x$, $R^y$, and $R^z$ are each as defined above.

Further, the group represented by $CR^4R^5R^6$ or the group represented by $CR^9R^{10}R^{11}$ may form an adamantyl group. For example, the following structure can be formed.

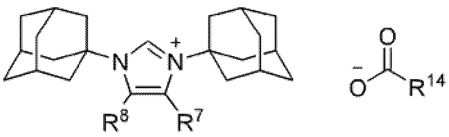 (5ay)

wherein $R^7$, $R^8$, and $R^{14}$ are each as defined above.

Examples of the imidazolium carboxylate (5) include

1,3-dimethylimidazolium acetate, 1-ethyl-3-methylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1-methyl-3-octadecylimidazolium acetate, 1-benzyl-3-methylimidazolium acetate, 1,3-dibutylimidazolium acetate, 1,3-di-tert-butylimidazolium acetate, 1-butyl-3-ethylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1-butyl-3-octadecylimidazolium acetate, 1-benzyl-3-butylimidazolium acetate, 1,3-dioctylimidazolium acetate, 1,3-bis(1,1,3,3-tetramethylbutyl)imidazolium acetate, 1-ethyl-3-octylimidazolium acetate, 1-ethyl-3-(1,1,3,3-tetramethylbutyl)imidazolium acetate, 1-octyl-3-(2-ethylhexyl)imidazolium acetate, 1-(1,1,3,3-tetramethylbutyl)-3-(2-ethylhexyl)imidazolium acetate, 1-dodecyl-3-octylimidazolium acetate, 1-dodecyl-3-(1,1,3,3-tetramethylbutyl)imidazolium acetate, 1-octyl-3-octadecylimidazolium acetate, 1-(1,1,3,3-tetramethylbutyl)-3-octadecylimidazolium acetate, 1-benzyl-3-octylimidazolium acetate, 1-benzyl-3-(1,1,3,3-tetramethylbutyl)imidazolium acetate, 1,3-bis(2-ethylhexyl)imidazolium acetate, 1-ethyl-3-(2-ethylhexyl)imidazolium acetate, 1-(2-ethylhexyl)-3-dodecylimidazolium acetate, 1-(2-ethylhexyl)-3-octadecylimidazolium acetate, 1-benzyl-3-(2-ethylhexyl)imidazolium acetate, 1,3-didodecylimidazolium acetate, 1-dodecyl-3-octadecylimidazolium acetate, 1-benzyl-3-dodecylimidazolium acetate, 1,3-dioctadecylimidazolium acetate, 1-benzyl-3-octadecylimidazolium acetate, 1,3-dibenzyl imidazolium acetate, 1,3-diisopropylimidazolium acetate, 1,3-dicyclohexylimidazolium acetate, 1,3-di(1-adamantyl)imidazolium acetate;

1,3-dimethylimidazolium 2-ethylhexanoate, 1-ethyl-3-methylimidazolium 2-ethylhexanoate, 1-butyl-3-methylimidazolium 2-ethylhexanoate, 1-methyl-3-octylimidazolium 2-ethylhexanoate, 1-methyl-3-(1,1,3,3-tetramethylbutyl)imidazolium 2-ethylhexanoate, 1-methyl-3-(2-ethylhexyl)imidazolium 2-ethylhexanoate, 1-dodecyl-3-methylimidazolium 2-ethylhexanoate, 1-methyl-3-octadecylimidazolium 2-ethylhexanoate, 1-benzyl-3-methylimidazolium 2-ethylhexanoate, 1,3-dibutylimidazolium 2-ethylhexanoate, 1,3-di-tert-butylimidazolium 2-ethylhexanoate, 1-butyl-3-ethylimidazolium 2-ethylhexanoate, 1-butyl-3-octylimidazolium 2-ethylhexanoate, 1-butyl-3-(1,1,3,3-tetramethylbutyl)imidazolium 2-ethylhexanoate, 1-butyl-3-(2-ethylhexyl)imidazolium 2-ethylhexanoate, 1-butyl-3-dodecylimidazolium 2-ethylhexanoate, 1-butyl-3-octadecylimidazolium 2-ethylhexanoate, 1-benzyl-3-butylimidazolium 2-ethylhexanoate, 1,3-dioctylimidazolium 2-ethylhexanoate, 1,3-bis(1,1,3,3-tetramethylbutyl)imidazolium 2-ethylhexanoate, 1-ethyl-3-octylimidazolium 2-ethylhexanoate, 1-ethyl-3-(1,1,3,3-tetramethylbutyl)imidazolium 2-ethylhexanoate, 1-octyl-3-(2-ethylhexyl)imidazolium 2-ethylhexanoate, 1-(1,1,3,3-tetramethylbutyl)-3-(2-ethylhexyl)imidazolium 2-ethylhexanoate, 1-dodecyl-3-octylimidazolium 2-ethylhexanoate, 1-dodecyl-3-(1,1,3,3-tetramethylbutyl)imidazolium 2-ethylhexanoate, 1-octyl-3-octadecylimidazolium 2-ethylhexanoate, 1-(1,1,3,3-tetramethylbutyl)-3-octadecylimidazolium 2-ethylhexanoate, 1-benzyl-3-octylimidazolium 2-ethylhexanoate, 1-benzyl-3-(1,1,3,3-tetramethylbutyl)imidazolium 2-ethylhexanoate, 1,3-bis(2-ethylhexyl)imidazolium 2-ethylhexanoate, 1-ethyl-3-(2-ethylhexyl)imidazolium 2-ethylhexanoate, 1-(2-ethylhexyl)-3-dodecylimidazolium 2-ethylhexanoate, 1-(2-ethylhexyl)-3-octadecylimidazolium 2-ethylhexanoate, 1-benzyl-3-(2-ethylhexyl)imidazolium 2-ethylhexanoate, 1,3-didodecylimidazolium 2-ethylhexanoate, 1-dodecyl-3-octadecylimidazolium 2-ethylhexanoate, 1-benzyl-3-dodecylimidazolium 2-ethylhexanoate, 1,3-dioctadecylimidazolium 2-ethylhexanoate, 1-benzyl-3-octadecylimidazolium 2-ethylhexanoate, 1,3-dibenzylimidazolium 2-ethylhexanoate, 1,3-diisopropylimidazolium 2-ethylhexanoate, 1,3-dicyclohexylimidazolium

2-ethylhexanoate, 1,3-di(1-adamantyl)imidazolium 2-ethylhexanoate;
1,3-dimethylbenzimidazolium acetate, 1,3-dimethylbenzimidazolium 2-ethylhexanoate, and the like.

Preferred among these are 1,3-dimethylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1-benzyl-3-methylimidazolium acetate, 1,3-dibutylimidazolium acetate, 1,3-di-tert-butylimidazolium acetate, 1-butyl-3-ethylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1-benzyl-3-butylimidazolium acetate, 1,3-dioctylimidazolium acetate, 1,3-bis(1,1,3,3-tetramethylbutyl)imidazolium acetate, 1-ethyl-3-octylimidazolium acetate, 1-ethyl-3-(1,1,3,3-tetramethylbutyl)imidazolium acetate, 1-octyl-3-(2-ethylhexyl)imidazolium acetate, 1-(1,1,3,3-tetramethylbutyl)-3-(2-ethylhexyl)imidazolium acetate, 1-dodecyl-3-octylimidazolium acetate, 1-dodecyl-3-(1,1,3,3-tetramethylbutyl)imidazolium acetate, 1-benzyl-3-octylimidazolium acetate, 1-benzyl-3-(1,1,3,3-tetramethylbutyl)imidazolium acetate, 1,3-bis(2-ethylhexyl)imidazolium acetate, 1-ethyl-3-(2-ethylhexyl)imidazolium acetate, 1-(2-ethylhexyl)-3-dodecylimidazolium acetate, 1-benzyl-3-(2-ethylhexyl)imidazolium acetate, 1,3-didodecylimidazolium acetate, 1-benzyl-3-dodecylimidazolium acetate, 1,3-dibenzyl imidazolium acetate, 1,3-diisopropylimidazolium acetate, 1,3-dicyclohexylimidazolium acetate, and 1,3-di(1-adamantyl)imidazolium acetate.

The imidazolium carboxylate (5) may be a commercial product. The imidazolium carboxylate (5) may be obtained by a known method, or produced by the method explained below.

The imidazolium carboxylate represented by Formula (5) is obtained by reacting a dicarbonyl compound represented by the following Formula (7), amine compounds represented by the following Formulas (8x) and (8y), formaldehyde, and a carboxylic acid represented by the following Formula (9).

Formula (7):

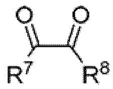

(7)

wherein $R^7$ and $R^8$ are as defined above.

Formula (8x):

$$G\text{-}NH_2 \qquad (8x)$$

wherein G is as defined above.

Formula (8y):

$$J\text{-}NH_2 \qquad (8y)$$

wherein J is as defined above.

Formula (9):

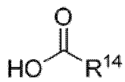

(9)

wherein $R^{14}$ is as defined above.

The dicarbonyl compound represented by Formula (7) (hereinafter referred to as the dicarbonyl compound (7)) is preferably, for example, glyoxal, diacetyl, 3,4-hexanedione, 2,3-pentanedione, 2,3-heptanedione, 5-methyl-2,3-hexanedione, 3-methyl-2,3-cyclopentanedione, 1,2-cyclohexanedione, 1-phenyl-1,2-propanedione, or dibenzoyl; more preferably glyoxal or diacetyl; and even more preferably glyoxal.

The amine compounds represented by Formulas (8x) and (8y) are preferably amine compounds represented by the following Formulas (8ax) and (8ay), respectively.

Formula (8ax):

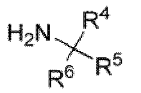

(8ax)

wherein $R^4$, $R^5$, and $R^6$ are each as defined above, and the group represented by $CR^4R^5R^6$ may be an adamantyl group.

Formula (8ay):

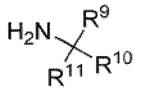

(8ay)

wherein $R^9$, $R^{10}$, and $R^{11}$ are each as defined above, and the group represented by $CR^9R^{10}R^{11}$ may be an adamantyl group.

Examples of the amine compound represented by Formula (8x) (hereinafter referred to as the amine compound (8x)) and the amine compound represented by Formula (8y) (hereinafter referred to as the amine compound (8y)) include at least one amine compound selected from the group consisting of methylamine, ethylamine, propylamine, isopropylamine, butylamine, tert-butylamine, hexylamine, octylamine, 1,1,3,3-tetramethylbutylamine, 2-ethylhexylamine, dodecylamine, tetradecylamine, hexadecylamine, octadecylamine, 1-adamantylamine, 2-methoxyethylamine, 2-ethoxyethylamine, 3-methoxypropylamine, 3-ethoxypropylamine, 3-propoxypropylamine, 3-isopropoxypropylamine, 3-butoxypropylamine, 3-(2-ethylhexyloxy)propylamine, allylamine, benzylamine, aniline, 2,6-diisopropylaniline, and 2,4,6-trimethylaniline; preferably methylamine, ethylamine, butylamine, tert-butylamine, hexylamine, octylamine, 1,1,3,3-tetramethylbutylamine, 2-ethylhexylamine, dodecylamine, octadecylamine, 1-adamantylamine, and benzylamine; more preferably methylamine, butylamine, octylamine, 2-ethylhexylamine, tert-butylamine, 1,1,3,3-tetramethylbutylamine, and 1-adamantylamine; and particularly preferably tert-butylamine and 1,1,3,3-tetramethylbutylamine.

Preferred examples of the carboxylic acid represented by Formula (9) (hereinafter referred to as the carboxylic acid (9)) include carboxylic acids, such as formic acid, acetic acid, propionic acid, butyric acid, pentanoic acid, hexanoic acid, heptanoic acid, octanoic acid, 2-ethylhexanoic acid, capric acid, lauric acid, tetradecyl acid, palmitic acid, octadecyl acid, cyclohexanoic acid, ethoxyacetic acid, propoxyacetic acid, 2-(2-methoxyethoxy)acetic acid, 2-(2-ethoxyethoxy)acetic acid, 2-(2-propoxyethoxy)acetic acid, 3-methoxypropanoic acid, 3-ethoxypropanoic acid, 3-(2-methoxyethoxy)propanoic acid, 3-(2-ethoxyethoxy)propanoic acid, 3-(2-propoxyethoxy)propanoic acid, 3-(3-methoxypropoxy)propanoic acid, 3-(3-ethoxypropoxy)propanoic acid, 3-(3-propoxypropoxy)propanoic acid, oleic acid, linoleic acid, sorbic acid, benzoic acid, phthalic acid, isophthalic acid, terephthalic acid, lactic acid, salicylic acid, and trifluoroacetic acid; more preferably formic acid, acetic acid, propionic acid, butyric acid, pentanoic acid, hexanoic acid, heptanoic acid, octanoic acid, and 2-ethylhexanoic acid; and particularly preferably acetic acid and 2-ethylhexanoic acid.

As the dicarbonyl compound (7), an aqueous solution or an alcohol solution, such as methanol or butanol, may be used as it is.

The amounts of the amine compound (8x) and amine compound (8y) (the amine compound (8x) and amine compound (8y) are hereinafter correctively referred to as the amine compound (8)) used are generally such that the amount of the amine compound (8) is 0.1 to 10 mol, and preferably 0.5 to 3 mol, per mol of the dicarbonyl compound (7). 2 mol of the amine compound (8) is reacted per mol of the dicarbonyl compound (7) to form 1 mol of the imidazolium carboxylate (5); however, for example, if the amount of the amine compound (8) is less than 2 mol, the dicarbonyl compound (7) (raw material) and a polymer of the dicarbonyl compound (7) will exist in addition to the target imidazolium carboxylate (5). Further, if more than 2 mol of the amine compound (8) is used per mol of the dicarbonyl compound (7), an excessive amount of the amine compound (8) will exist in addition to the target imidazolium carboxylate (5). The amidate compound (2) can also be obtained by using the imidazolium carboxylate (5) in which such compounds other than the imidazolium carboxylate (5) coexist.

The ratio of the amine compound (8x) to the amine compound (8y) (amine compound (8x):amine compound (8y)) is not particularly limited, and is within the range of 0:100 to 100:0. When the ratio of the amine compound (8x) to the amine compound (8y) is 0:100 or 100:0, G=J holds. In cases other than G=J, i.e., when the ratio of the amine compound (8x) to the amine compound (8y) is not 0:100 or 100:0, the imidazolium carboxylate represented by Formula (5) can be a mixture of compounds represented by the following Formulas (5-1), (5-2), and (5-3):

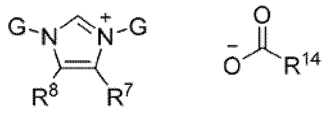 (5-1)

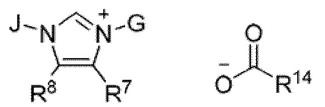 (5-2)

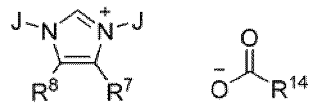 (5-3)

wherein in Formulas (5-1), (5-2), and (5-3), $R^7$, $R^8$, $R^{14}$, G, and J are each as defined above.

The ratio of the compounds represented by formulas (5-1), (5-2), and (5-3) in the mixture varies depending on the ratio of the amine compound (8x) and amine compound (8y) used in the reaction. The compounds represented by formulas (5-1), (5-2), and (5-3) are all included in the imidazolium carboxylate (5).

As formaldehyde, an aqueous solution or an alcohol solution, such as methanol or butanol, may be used as it is. The amount of formaldehyde used is generally 0.1 to 10 mol, and preferably 0.5 to 5.0 mol, per mol of the dicarbonyl compound (7) .

The amount of the carboxylic acid (9) used is generally 0.1 to 10 mol, preferably 0.5 to 2 mol, and more preferably 1 to 1.5 mol, per mol of the dicarbonyl compound (7).

The optimal reaction temperature varies depending on the raw materials, solvents, etc. used, but is generally -10°C or higher, and preferably 0°C to 100°C. The reaction time is generally 0.1 to 48 hours, and preferably 0.5 to 12 hours.

A solvent may or may not be used. When a solvent is used, the solvent used is not particularly limited, as long as it does not affect the reaction. Specific examples of solvents include aromatic hydrocarbons, such as toluene, benzene, and xylene; aliphatic or alicyclic hydrocarbons, such as methylcyclohexane, cyclohexane, hexane, heptane, and octane; halogenated hydrocarbons, such as dichloromethane, chloroform, carbon tetrachloride, and 1,2-dichloroethane; ethers, such as diethyl ether, tetrahydrofuran, and 1,4-dioxane; lower alcohols, such as methanol and ethanol; N,N-dimethylformamide, acetonitrile, water, and the like. Preferred among these are aromatic hydrocarbons, lower alcohols, and water; and particularly preferred are toluene and water. The solvents can be used as a mixture of two or more, if necessary.

The amount of solvent used is generally 50 parts by mass or less, and preferably 0.1 to 10 parts by mass, per part by mass of the dicarbonyl compound (7).

The reaction may be performed, if necessary, in an inert gas atmosphere, such as nitrogen, argon, or helium, which do not affect the reaction.

After completion of the reaction, the imidazolium carboxylate (5) can be isolated, for example, by removing impurities (e.g., unreacted raw materials) by washing with an organic solvent, or concentrating the reaction liquid, and may be purified by recrystallization, etc., if necessary.

The ester carbonate represented by Formula (6) (hereinafter referred to as the ester carbonate (6)) is explained.

In Formula (6), $R^{15}$ and $R^{16}$ are the same or different, and each represents a $C_1$-$C_6$ hydrocarbon group, preferably a $C_1$-$C_4$ hydrocarbon group, and particularly preferably a methyl group. $R^{15}$ and $R^{16}$ may form a ring structure together with the oxygen atoms to which they are bonded. Examples of $C_1$-$C_6$ hydrocarbon groups include methyl, ethyl, propyl, isopropyl, butyl, sec-butyl, tert-butyl, pentyl, hexyl, and phenyl groups. Specific examples of the ester carbonate (6) include dialkyl carbonates, such as dimethyl carbonate, diethyl carbonate, dipropyl carbonate, dibutyl carbonate, dipentyl carbonate, and dihexyl carbonate; and cyclic alkylene carbonates, such as ethylene carbonate, propylene carbonate, and butylene carbonate. Preferred among these are dimethyl carbonate, diethyl carbonate, dipropyl carbonate, and dibutyl carbonate; and particularly preferred is dimethyl carbonate.

The amount of the ester carbonate (6) used is generally 1 mol or more, and preferably 1 to 6 mol, per mol of the remaining carboxylic acid (9). When the imidazolium carboxylate (5) contains water together with the carboxylic acid (9), the water reacts with the ester carbonate; thus, it is preferable to use the ester carbonate generally in excess of 1 mol or more, and preferably 1 to 6 mol, per mol of the total of the carboxylic acid (9) and water contained in the imidazolium carboxylate (5). The carboxylic acid (9) can be converted into a corresponding ester compound at a reaction temperature of 30 to 100°C for a reaction time of 1 to 8 hours. The converted ester compound can be removed, for example, by washing with an organic solvent or concentration of the reaction liquid, thereby removing the carboxylic acid (9) from the imidazolium carboxylate (5). Further, the target amidate compound (2) can also be obtained even when using the imidazolium carboxylate (5) containing an ester compound converted by the ester carbonate.

The amount of the ester carbonate (6) used is generally 1 mol or more, and preferably 1 to 6 mol, per mol of the imidazolium carboxylate (5). When the imidazolium carboxylate (5) contains excess carboxylic acid and water, they react with the ester carbonate (6); thus, it is preferable to use the ester carbonate (6) generally in excess of 1 mol or more, and preferably 1 to 6 mol, per mol of the total of the excess carboxylic acid and water in the imidazolium carboxylate (5).

In Reaction 1, a solvent may or may not be used. When a solvent is used, the solvent used is not particularly limited, as long as it does not affect the reaction. Specific examples of solvents include monovalent alcohol solvents, such as methanol, ethanol, propanol, butanol, pentanol, hexanol, 1-methoxy-2-propanol, and ethoxyethanol; polyol solvents, such as ethylene glycol, propylene glycol, and diethylene glycol; glycol monoalkyl ether solvents, such as dipropylene glycol monobutyl ether, dipropylene glycol monomethyl ether, and tripropylene glycol monomethyl ether; aromatic hydrocarbon solvents, such as toluene, benzene, and xylene; aliphatic hydrocarbon solvents, such as methylcyclohexane, cyclohexane, hexane, heptane, and octane; ester solvents, such as ethyl acetate and butyl acetate; ketone solvents, such as methyl ethyl ketone and 4-methyl-2-pentanone; and the like. Preferred among these are monovalent alcohol solvents, and particularly preferred is methanol. The amount of solvent used is generally 50 parts by mass or less, and preferably 10 parts by mass or less, per part by mass of the imidazolium carboxylate (5).

In Reaction 1, the optimal reaction temperature can vary depending on the raw materials, solvents, etc., used, and is generally room temperature or higher, and preferably 20 to 200°C. In the present specification, room temperature generally means about 20°C. The reaction time is generally 0.1 to 48 hours, and preferably 1 to 24 hours.

In Reaction 1, the reaction may be performed, if necessary, in an inert gas atmosphere, such as nitrogen, argon, or helium, which do not affect the reaction.

After completion of the reaction, the reaction liquid may be concentrated to remove the solvent, if necessary.

When the ester carbonate (6) and the solvent remain in the reaction liquid, the reaction liquid can be concentrated to remove the ester carbonate (6) and the solvent.

Reaction 2 is explained.

The reaction product obtained in Reaction 1 (hereinafter also referred to as the reaction product (R)) is reacted with an isocyanate compound represented by Formula (10) to produce the amidate compound (2).

The isocyanate compound represented by Formula (10) (hereinafter referred to as the isocyanate compound (10)) is explained.

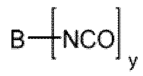
(10)

wherein B and y are each as defined above.

In the present invention, the isocyanate compound (10) is preferably a compound represented by any of the following Formulas (10-1), (10-2), and (10-3).

Formula (10-1):

$$R^{12}\text{-NCO} \qquad (10\text{-}1)$$

wherein $R^{12}$ is as defined above.

Formula (10-2):

$$OCN\text{-}R^{13}\text{-NCO} \qquad (10\text{-}2)$$

wherein $R^{13}$ is as defined above.

Formula (10-3):

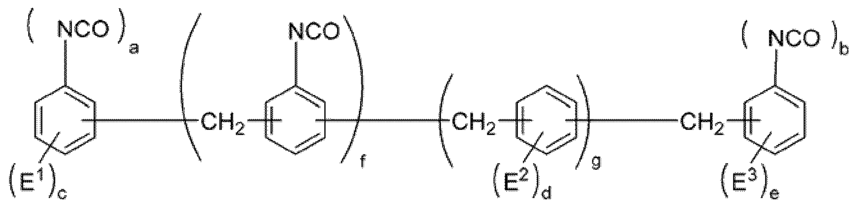
(10-3)

wherein $E^1$, $E^2$, $E^3$, a, b, c, d, e, f, and g are each as defined above.

In the present invention, a polymer such as polymethylene polyphenyl polyisocyanate (polymeric MDI) can also be used as the isocyanate compound (10).

Specific examples of the isocyanate compound (10) are shown below. However, the present invention is not limited thereto. In the following specific examples, Me represents a methyl group, iPr represents an isopropyl group, Bu represents an n-butyl group, Oct represents an n-octyl group, and MeO represents a methoxy group.

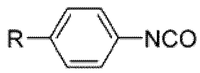

| R | |
|---|---|
| Me | (10-1-1) |
| iPr | (10-1-2) |
| Bu | (10-1-3) |
| Oct | (10-1-4) |
| MeO | (10-1-5) |
| Cl | (10-1-6) |

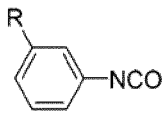

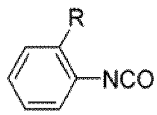

| R | | | |
|---|---|---|---|
| Me | (10-1-7) | Me | (10-1-13) |
| iPr | (10-1-8) | iPr | (10-1-14) |
| Bu | (10-1-9) | Bu | (10-1-15) |
| 0ct | (10-1-10) | Oct | (10-1-16) |
| Me0 | (10-1-11) | Me0 | (10-1-17) |
| Cl | (10-1-12) | Cl | (10-1-18) |

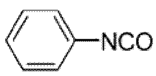 (10-1-19)

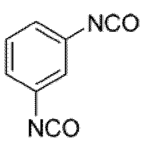 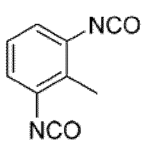 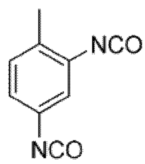 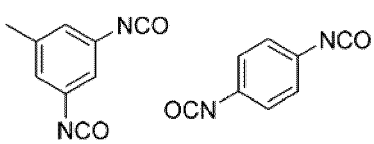 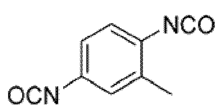

(10-2-1) (10-2-2) (10-2-3) (10-2-4) (10-2-5) (10-2-6)

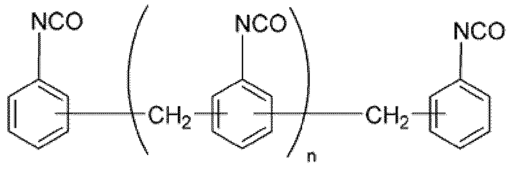

(10-3-1)

wherein n is as defined above.

The isocyanate compound (10) is preferably a compound represented by Formula (10-1-5), (10-1-19), (10-2-3), or (10-3-1), and more preferably a compound represented by Formula (10-1-19) or (10-3-1).

The isocyanate compounds (10) may be used singly or as a mixture of two or more.

In Reaction 2, the amount of the isocyanate compound (10) used is generally such that the amount of isocyanate groups in the isocyanate compound (10) is 0.8 mol or more, and preferably 1 to 3 mol, per mol of the imidazolium carboxylate (5) .

The reaction temperature is not particularly limited, but is generally -10°C or higher, preferably 0 to 200°C, and more preferably 20 to 150°C. The reaction time is generally 0.1 to 48 hours, and preferably 0.5 to 24 hours.

When a solvent is used, examples of solvents include aromatic hydrocarbon solvents, such as toluene, benzene, and xylene; aliphatic hydrocarbon solvents, such as methylcyclohexane, cyclohexane, hexane, heptane, and octane; halogenated hydrocarbon solvents, such as butyl chloride and 1,2-dichloroethane; halogenated aromatic hydrocarbon solvents, such as chlorobenzene; ester solvents, such as ethyl acetate and butyl acetate; ketone solvents, such as methyl ethyl ketone and 4-methyl-2-pentanone; and the like. Preferred among these are aromatic hydrocarbon solvents, halogenated aromatic hydrocarbon solvents, ester solvents, and ketone solvents; and particularly preferred are toluene, xylene, chlorobenzene, butyl acetate, and 4-methyl-2-pentanone. The solvents can be used as a mixture of two or more, if necessary. When the reaction product (R) contains the solvent used in Reaction 1, this solvent can also be used in Reaction 2 as a solvent; however, a solvent mentioned above may be further added. In this case, a solvent different from that of Reaction 1 may be used.

The amount of solvent used is generally 50 parts by mass or less, and preferably 0.1 parts by mass or more and 35 parts by mass or less, per part by mass of the imidazolium carboxylate (5).

The reaction may be performed, if necessary, in an inert gas atmosphere, such as nitrogen, argon, or helium, which do not affect the reaction.

After completion of the reaction, the amidate compound (2) can be obtained by removing the solvent by concentrating or filtering the reaction liquid, and may be purified by recrystallization, column separation, etc., if necessary.

In the present invention, the blocking agent dissociation catalyst for blocked isocyanate compounds (hereinafter referred to as the blocking agent dissociation catalyst) is a catalyst for dissociating a blocking agent that is blocking isocyanate groups of a blocked isocyanate compound to form an isocyanate compound, and promoting the reaction with a compound having an isocyanate-reactive group, described later. The amidate compound (2) functions as a blocking agent dissociation catalyst. In particular, the amidate compound represented by Formula (2a) more preferably functions as a blocking agent dissociation catalyst. The amidate compound represented by Formula (2a) also functions as a blocking agent dissociation catalyst for blocked isocyanate compounds formed by using a known blocking agent such as methyl ethyl ketoxime, but is particularly effective as a blocking agent dissociation catalyst for the fluoroalcohol-blocked isocyanate compound (BI).

Further, the amidate compound represented by Formula (2a) more preferably functions as a blocking agent dissociation catalyst for the blocked isocyanate compound (1a), and promotes the reaction with a compound having an isocyanate-reactive group at a lower temperature or in a shorter period of time than conventional blocking agent dissociation catalysts. Therefore, a blocked isocyanate composition comprising the amidate compound represented by Formula (2a) and the blocked isocyanate compound (1a) particularly has excellent low-temperature curing properties when formed as the thermosetting resin composition of the present invention, described later.

In the blocked isocyanate composition of the present invention, the mixing ratio of the fluoroalcohol-blocked isocyanate compound (BI) and the amidate compound (2) is determined by the required physical properties, and is not particularly limited. The mixing ratio is generally within the following range: [amidate groups (mol) in the amidate compound (2)]/[effective isocyanate groups (mol) in the fluoroalcohol-blocked isocyanate compound (BI)] = 0.001 to 0.5, and preferably 0.01 to 0.1. The amidate groups in the amidate compound (2) refer to functional groups represented by the following Formula (A), and the effective isocyanate groups in the fluoroalcohol-blocked isocyanate compound (BI) refer to isocyanate groups that are regenerated when the fluoroalcohol compound (F), which is the blocking agent, is dissociated from the fluoroalcohol-blocked isocyanate compound (BI).

Formula (A):

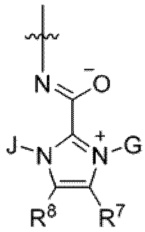

(A)

wherein G, J, $R^7$, and $R^8$ are each as defined above.

In the blocked isocyanate composition of the present invention, known catalysts for polyurethane production, additives, pigments, solvents, and the like that are commonly used in this technical field can be used, if necessary.

Known catalysts for polyurethane production are not particularly limited. Examples include tin compounds, such as dibutyltin dilaurate, dibutyltin di-2-ethylhexanate, dioctyltin dilaurate, dibutyltin diacetate, dibutyltin dioxide, dioctyltin dioxide, tin acetylacetonate, tin acetate, tin octylate, and tin laurate; bismuth compounds, such as bismuth octylate, bismuth naphthenate, and bismuth acetylacetonate; titanium compounds, such as tetra-n-butyl titanate, tetraisopropyl titanate, and titanium terephthalate; tertiary amine compounds, such as triethylamine, N,N,N',N'-tetramethylethylenediamine, N,N,N',N'-tetramethylpropylenediamine, N,N,N',N",N"-pentamethyldiethylenetriamine, N,N,N',N",N"-pentamethyldipropylenetriamine, N,N,N',N'-tetramethylguanidine, 1,3,5-tris(N,N-dimethylaminopropyl)hexahydro-S-triazine, 1,4-diazabicyclo[2.2.2]octane (DABCO), 1,8-diazabicyclo[5.4.0]undecene-7, triethylenediamine, N,N,N',N'-tetramethylhexamethylenediamine, N-methyl-N'-(2-dimethylaminoethyl)piperazine, N,N'-dimethylpiperazine, dimethylcyclohexylamine, N-methylmorpholine, N-ethylmorpholine, bis(2-dimethylaminoethyl)ether, 1-methylimidazole, 1,2-dimethylimidazole, 1-isobutyl-2-methylimidazole, and 1-dimethylaminopropylimidazole; and quaternary ammonium salt compounds, such as tetraalkylammonium halides (e.g., tetramethylammonium chloride), tetraalkylammonium hydroxides (e.g., tetramethylammonium hydroxide salts), tetraalkylammonium organic acid salts (e.g., tetramethylammonium-2-ethylhexanoate, 2-hydroxypropyl trimethylammonium formate, and 2-hydroxypropyl trimethylammonium-2-ethylhexanoate).

Additives are not particularly limited. Examples include hindered amine-based, benzotriazole-based, and benzophenone-based UV absorbers; perchlorate-based and hydroxylamine-based coloration inhibitors; hindered phenol-based, phosphorus-based, sulfur-based, and hydrazide-based antioxidants; tin-based, zinc-based, and amine-based urethanization catalysts; leveling agents, rheology control agents, pigment dispersants, and the like.

Pigments are not particularly limited. Examples include organic pigments, such as quinacridone-based, azo-based, and phthalocyanine-based pigments; inorganic pigments, such as titanium oxide, barium sulfate, calcium carbonate, and silica; and other pigments, such as carbon-based pigments, metal foil pigments, and rust-preventive pigments.

Solvents are not particularly limited. Examples include hydrocarbons, such as benzene, toluene, xylene, cyclohexane, mineral spirit, and naphtha; ketones, such as acetone, methyl ethyl ketone, and methyl isobutyl ketone; esters, such as ethyl acetate, butyl acetate, and cellosolve acetate; alcohols, such as methanol, ethanol, 2-propanol, butanol, 2-methoxyethanol, 2-ethoxyethanol, and 2-butoxyethanol; polyhydric alcohols, such as ethylene glycol, propylene glycol, diethylene glycol, polyethylene glycol, and glycerol; water; and the like. These solvents may be used singly or in combination of two or more.

The thermosetting resin composition of the present invention is explained.

The thermosetting resin composition of the present invention comprises the blocked isocyanate composition of the present invention and a compound having an isocyanate-reactive group.

Examples of the compound having an isocyanate-reactive group include compounds having two or more active hydrogen groups, such as polyols, polyamines, and alkanolamines. These compounds having an isocyanate-reactive group may be a mixture of two or more.

In the present invention, polyols are compounds having two or more hydroxyl groups. Examples include polyester polyols, polyether polyols, acrylic polyols, polyolefin polyols, fluorine polyols, and the like. Preferred polyols among these are acrylic polyols in terms of weather resistance, chemical resistance, and hardness. Alternatively, polyols preferred in terms of mechanical strength and oil resistance are polyester polyols. These polyols may be a mixture of two or more.

Examples of polyether polyols include active hydrogen compounds, such as aliphatic amine polyols, aromatic amine polyols, Mannich polyols, polyhydric alcohols, polyhydric phenols, and bisphenols; compounds obtained by adding alkylene oxides to these active hydrogen compounds; and the like. These polyether polyols may be a mixture of two or more.

Examples of aliphatic amine polyols include alkylenediamine-based polyols and alkanolamine-based polyols. These polyol compounds are polyfunctional polyol compounds having terminal hydroxyl groups obtained by the ring-opening addition of at least one cyclic ether, such as ethylene oxide or propylene oxide, using alkylenediamine or alkanolamine as an initiator. As the alkylenediamine, known compounds can be used without limitation. Specifically, $C_2$-$_8$ alkylenediamines, such as ethylenediamine, propylenediamine, butylenediamine, hexamethylenediamine, and neopentyldiamine, are preferably used. These aliphatic amine polyols may be a mixture of two or more.

Aromatic amine polyols are polyfunctional polyether polyol compounds having terminal hydroxyl groups obtained by the ring-opening addition of at least one cyclic ether, such as ethylene oxide or propylene oxide, using an aromatic diamine as an initiator. As the initiator, a known aromatic diamine can be used without limitation. Specific examples include 2,4-toluenediamine, 2,6-toluenediamine, diethyltoluenediamine, 4,4'-diaminodiphenylmethane, p-phenylenediamine, o-phenylenediamine, naphthalenediamine, and the like. Among these, toluenediamine (2,4-toluenediamine, 2,6-toluenediamine, or a mixture thereof) is particularly preferably used. These aromatic amine polyols may be a mixture of two or more.

Mannich polyols are active hydrogen compounds obtained by the Mannich reaction of phenol and/or an alkyl-substituted derivative thereof, formaldehyde, and alkanolamine, or polyol compounds obtained by the ring-opening addition polymerization of the active hydrogen compounds with at least one of ethylene oxide and propylene oxide. These Mannich polyols may be a mixture of two or more.

Examples of polyhydric alcohols include dihydric alcohols (e.g., ethylene glycol, propylene glycol, 1,4-butanediol, 1,6-hexanediol, diethylene glycol, triethylene glycol, dipropylene glycol, and neopentyl glycol), trihydric or higher alcohols (e.g., glycerol, trimethylolpropane, pentaerythritol, methylglucoside, sorbitol, and sucrose), and the like. These polyhydric alcohols may be a mixture of two or more.

Examples of polyhydric phenols include pyrogallol, hydroquinone, and the like. These polyhydric phenols may be a mixture of two or more.

Examples of bisphenols include bisphenol A, bisphenol S, bisphenol F, low-condensates of phenols and formaldehyde, and the like. These bisphenols may be a mixture of two or more.

Polyester polyols can be obtained, for example, by the condensation reaction of a single dibasic acid or a mixture of two or more dibasic acids with a single polyhydric alcohol or a mixture of two or more polyhydric alcohols.

Examples of dibasic acids include carboxylic acids, such as succinic acid, adipic acid, dimer acid, maleic anhydride, phthalic anhydride, isophthalic acid, terephthalic acid, and 1,4-cyclohexanedicarboxylic acid; and the like.

Examples of polyhydric alcohols include ethylene glycol, propylene glycol, diethylene glycol, 1,4-butanediol, neopentyl glycol, 1,6-hexanediol, trimethylpentanediol, cyclohexanediol, trimethylolpropane, glycerol, pentaerythritol, 2-methylolpropanediol, ethoxylated trimethylolpropane, and the like.

As a specific method for producing polyester polyols, for example, the condensation reaction can be carried out by mixing the above components, and heating the mixture at about 160 to 220°C. Alternatively, for example, polycaprolactones obtained by the ring-opening polymerization of lactones, such as ε-caprolactone, with polyhydric alcohols can also be used as polyester polyols.

These polyester polyols can be modified by using, for example, aromatic diisocyanates, aliphatic diisocyanates, alicyclic diisocyanates, and polyisocyanates obtained from them. Among these, in terms of weather resistance, yellowing resistance, etc., polyester polyols are preferably modified by using aliphatic diisocyanates, alicyclic diisocyanates, and polyisocyanates obtained from them.

When the thermosetting resin composition of the present embodiment is used as an aqueous-based paint, some carboxylic acids derived from the dibasic acid etc. in the polyester polyol can be allowed to remain and neutralized with a base, such as amine or ammonia, thereby forming the polyester polyol into a water-soluble or water-dispersible resin.

Polyether polyols can be obtained, for example, by any of the following methods (1) to (3).

(1) A method of performing random or block addition of an alkylene oxide alone or a mixture of alkylene oxides to a polyhydroxy compound alone or a mixture of polyhydroxy compounds using a catalyst to obtain polyether polyols.

Examples of catalysts include alkali metal hydroxides (lithium hydroxide, sodium hydroxide, potassium hydroxide, etc.), strong base catalysts (alcoholates, alkylamines, etc.), composite metal cyanide compound complexes (metal porphyrins, zinc hexacyanocobaltate complexes, etc.), and the like.

Examples of alkylene oxides include ethylene oxide, propylene oxide, butylene oxide, cyclohexene oxide, styrene oxide, and the like.

(2) A method of reacting polyamine compounds with alkylene oxides to obtain polyethers polyols.

Examples of polyamine compounds include ethylene diamines and the like.

Examples of alkylene oxides include those mentioned in (1) .

(3) A method of polymerizing acrylamide etc. using the polyether polyols obtained in (1) or (2) as media to obtain so-called polymer polyols.

Examples of polyhydroxy compounds include the following (i) to (vi).

(i) diglycol, ditrimethylolpropane, pentaerythritol, dipentaerythritol, etc.
(ii) sugar alcohol-based compounds, such as erythritol, D-threitol, L-arabinitol, ribitol, xylitol, sorbitol, mannitol, galactitol, and rhamnitol
(iii) monosaccharides, such as arabinose, ribose, xylose, glucose, mannose, galactose, fructose, sorbose, rhamnose, fucose, and ribodesose
(iv) disaccharides, such as trehalose, sucrose, maltose, cellobiose, gentiobiose, lactose, and melibiose
(v) trisaccharides, such as raffinose, gentianose, and melezitose
(vi) tetrasaccharides, such as stachyose

Acrylic polyols can be obtained, for example, by polymerizing polymerizable monomers having one or more active hydrogens per molecule, or by copolymerizing polymerizable monomers having one or more active hydrogens per molecule with other monomers copolymerizable with the polymerizable monomers, if necessary.
Examples of polymerizable monomers having one or more active hydrogens per molecule include the following (i) to (vi). These may be used singly or in combination of two or more.

(i) acrylic acid esters having active hydrogen, such as 2-hydroxyethyl acrylate, 2-hydroxypropyl acrylate, and 2-hydroxybutyl acrylate
(ii) methacrylic acid esters having active hydrogen, such as 2-hydroxyethyl methacrylate, 2-hydroxypropyl methacrylate, 2-hydroxybutyl methacrylate, 3-hydroxypropyl methacrylate, and 4-hydroxybutyl methacrylate
(iii) (meth)acrylic acid esters having polyvalent active hydrogen, such as (meth)acrylic acid monoesters of triols, such as glycerol and trimethylolpropane
(iv) monoethers of polyether polyols (e.g., polyethylene glycol, polypropylene glycol, and polybutylene glycol) with the above (meth)acrylic acid esters having active hydrogen
(v) adducts of glycidyl (meth)acrylate with monobasic acids (e.g., acetic acid, propionic acid, and p-tert-butyl benzoic acid)
(vi) adducts obtained by the ring-opening polymerization of lactones (e.g., ε-caprolactam and γ-valerolactone) with the active hydrogen of the above (meta)acrylic acid esters having active hydrogen

Examples of monomers copolymerizable with the above polymerizable monomers include the following (i) to (iv). These may be used singly or in combination of two or more.

(i) (meth)acrylic acid esters, such as methyl acrylate, ethyl acrylate, isopropyl acrylate, n-butyl acrylate, 2-ethylhexyl acrylate, methyl methacrylate, ethyl methacrylate, isopropyl methacrylate, n-butyl methacrylate, isobutyl methacrylate, n-hexyl methacrylate, cyclohexyl methacrylate, lauryl methacrylate, and glycidyl methacrylate
(ii) unsaturated carboxylic acids, such as acrylic acid, methacrylic acid, maleic acid, and itaconic acid; and unsaturated amides, such as acrylamide, N-methylolacrylamide, and diacetoneacrylamide
(iii) vinyl monomers having a hydrolyzable silyl group, such as vinyl trimethoxysilane, vinyl methyl dimethoxysilane, and γ-(meth)acrylopropyltrimethoxysilane
(iv) other polymerizable monomers, such as styrene, vinyl toluene, vinyl acetate, acrylonitrile, and dibutyl fumarate

As a specific method for producing acrylic polyols, for example, the above monomer components are subjected to solution polymerization in the presence of a known radical polymerization initiator, such as a peroxide or an azo compound, optionally followed by dilution with an organic solvent etc., thereby obtaining acrylic polyols.
When the thermosetting resin composition of the present embodiment is used as an aqueous-based paint, aqueous-based acrylic polyols can be produced by solution polymerization of the above monomer components, and conversion to an aqueous layer, or by using a known method, such as emulsion polymerization. In that case, the acidic portions of carboxylic acid-containing monomers and sulfonic acid-containing monomers, such as acrylic acid and methacrylic acid, can be neutralized with amine or ammonia to make acrylic polyols water-soluble or water-dispersible.
Examples of polyolefin polyols include polybutadiene having two or more hydroxyl groups, hydrogenated polybutadiene having two or more hydroxyl groups, hydrogenated polyisoprene having two or more hydroxyl groups, and the like.
In the polyolefin polyol, the number of hydroxyl groups is preferably three because higher coating film strength can be obtained.
In the present specification, "fluorine polyols" refer to polyols containing fluorine in the molecule. Specific examples of fluorine polyols include the copolymers of fluoroolefin, cyclovinyl ether, hydroxyalkyl vinyl ether, and vinyl monocarboxylate disclosed in JPS57-34107A, JPS61-275311A, etc.
The lower limit of the hydroxyl value of the polyol is preferably 10 mgKOH/g or more, more preferably 20 mgKOH/g or more, and even more preferably 30 mgKOH/g or more.

On the other hand, the upper limit of the hydroxyl value of the polyol is not particularly limited, and may be, for example, 200 mgKOH/g or less.

Specifically, the hydroxyl value of the polyol is preferably 10 mgKOH/g or more and 200 mgKOH/g or less, more preferably 20 mgKOH/g or more and 200 mgKOH/g or less, and even more preferably 30 mgKOH/g or more and 200 mgKOH/g or less.

Further, the acid value of the polyol is preferably 0 mgKOH/g or more and 30 mgKOH/g or less.

The hydroxyl value and acid value can be measured according to JIS K1557.

The molar equivalent ratio (NCO/OH) of isocyanate groups in the blocked isocyanate composition to hydroxyl groups in the polyol is preferably 0.2 or more and 5.0 or less, more preferably 0.4 or more and 3.0 or less, and even more preferably 0.5 or more and 2.0 or less.

Usable polyamines are those having two or more primary amino groups or secondary amino groups per molecule. Preferred among these are those having three or more such amino groups per molecule.

Specific examples of polyamines include diamines, such as ethylenediamine, propylenediamine, butylenediamine, triethylenediamine, hexamethylenediamine, 4,4'-diaminodicyclohexylmethane, piperazine, 2-methylpiperazine, and isophoronediamine; chain polyamines having three or more amino groups, such as bishexamethylenetriamine, diethylenetriamine, triethylenetetramine, tetraethylenepentamine, pentamethylenehexamine, and tetrapropylenepentamine; and cyclic polyamines, such as 1,4,7,10,13,16-hexaazacyclooctadecane, 1,4,7,10-tetraazacyclodecane, 1,4,8,12-tetraazacyclopentadecane, and 1,4,8,11-tetraazacyclotetradecane.

Alkanolamines refer to compounds having an amino group and a hydroxyl group per molecule. Examples of alkanolamines include monoethanolamine, diethanolamine, aminoethylethanolamine, N-(2-hydroxypropyl)ethylenediamine, mono-, di-(n- or iso-)propanolamine, ethylene glycol-bis-propylamine, neopentanolamine, methylethanolamine, and the like.

The thermosetting resin composition of the present embodiment may contain, if necessary, melamine-based curing agents, such as complete alkyl type, methylol type, and alkylamino group type alkyl.

The thermosetting resin composition of the present embodiment may contain an organic solvent.

Further, the compound having an isocyanate-reactive group and the blocked isocyanate composition described above may contain an organic solvent.

Preferable organic solvents are those that are compatible with the blocked isocyanate composition.

Specific examples of organic solvents include hydrocarbons, such as benzene, toluene, xylene, cyclohexane, mineral spirit, and naphtha; ketones, such as acetone, methyl ethyl ketone, and methyl isobutyl ketone; esters, such as ethyl acetate, butyl acetate, and cellosolve acetate; alcohols, such as methanol, ethanol, 2-propanol, butanol, 2-methoxyethanol, 2-ethoxyethanol, and 2-butoxyethanol; polyhydric alcohols, such as ethylene glycol, propylene glycol, diethylene glycol, polyethylene glycol, and glycerol; water; and the like. These solvents may be used singly or in combination of two or more.

Further, the thermosetting resin composition of the present embodiment can be used as an aqueous thermosetting resin composition dissolved or dispersed in water. When the thermosetting resin composition of the present invention is used as an aqueous thermosetting resin composition, in order to improve the compatibility of the thermosetting resin composition, surfactants, solvents that tend to be miscible with water, etc. may be used for the blocked isocyanate composition of the present invention. Examples of surfactants include anionic surfactants, such as aliphatic soaps, rosin acid soaps, alkyl sulfonates, dialkylaryl sulfonates, alkyl sulfosuccinates, polyoxyethylene alkyl sulfates, and polyoxyethylene alkylaryl sulfates; and nonionic surfactants, such as polyoxyethylene alkyl ethers, polyoxyethylene alkyl aryl ethers, and polyoxyethylene oxypropylene block copolymers. Examples of solvents that tend to be miscible with water include diethylene glycol dimethyl ether, diethylene glycol diethyl ether, propylene glycol monomethyl ether acetate, propylene glycol monomethyl ether, propylene glycol dimethyl ether, dipropylene glycol dimethyl ether, isobutanol, butyl glycol, N-methylpyrrolidone, butyl diglycol, butyl diglycol acetate, and the like.

Preferred among the above solvents are diethylene glycol dimethyl ether, diethylene glycol diethyl ether, propylene glycol monomethyl ether, propylene glycol dimethyl ether, dipropylene glycol dimethyl ether, isobutanol, butyl glycol, N-methylpyrrolidone, and butyl diglycol; and more preferred are diethylene glycol dimethyl ether, diethylene glycol diethyl ether, propylene glycol dimethyl ether, and dipropylene glycol dimethyl ether. These solvents may be used singly or in combination of two or more. Ester solvents, such as ethyl acetate, n-butyl acetate, and cellosolve acetate, are not preferred because the solvents themselves may hydrolyze during storage.

In the thermosetting resin composition of the present invention, the mixing ratio of the blocked isocyanate composition and the compound having an isocyanate-reactive group is determined by the required physical properties, and is not particularly limited. The mixing ratio is generally within the following range: [the amount of effective isocyanate groups (mol) in the blocked isocyanate compound in the blocked isocyanate composition]/[the amount of active hydrogen groups (mol) in the compound having an isocyanate-reactive group] = 0.2 to 5, and preferably 0.5 to 3. The effective isocyanate groups in the blocked isocyanate compound refer to isocyanate groups that are regenerated when the blocking agent is dissociated from the blocked isocyanate compound.

In the thermosetting resin composition of the present invention, known catalysts for polyurethane production, additives, pigments, and the like that are commonly used in this technical field can be used, if necessary. These may be used as a mixture with known blocked polyisocyanates.

Known catalysts for polyurethane production are not particularly limited. Examples include tin compounds, such as dibutyltin dilaurate, dibutyltin di-2-ethylhexanate, dioctyltin dilaurate, dibutyltin diacetate, dibutyltin dioxide, dioctyltin dioxide, tin acetylacetonate, tin acetate, tin octylate, and tin laurate; bismuth compounds, such as bismuth octylate, bismuth naphthenate, and bismuth acetylacetonate; titanium compounds, such as tetra-n-butyl titanate, tetraisopropyl titanate, and titanium terephthalate; tertiary amine compounds, such as triethylamine, N,N,N',N'-tetramethylethylenediamine, N,N,N',N'-tetramethylpropylenediamine, N,N,N',N",N"-pentamethyldiethylenetriamine, N,N,N',N",N"-pentamethyldipropylenetriamine, N,N,N',N'-tetramethylguanidine, 1,3,5-tris(N,N-dimethylaminopropyl)hexahydro-S-triazine, 1,4-diazabicyclo[2.2.2]octane (DABCO), 1,8-diazabicyclo[5.4.0]undecene-7, triethylenediamine, N,N,N',N'-tetramethylhexamethylenediamine, N-methyl-N'-(2-dimethylaminoethyl)piperazine, N,N'-dimethylpiperazine, dimethylcyclohexylamine, N-methylmorpholine, N-ethylmorpholine, bis(2-dimethylaminoethyl)ether, 1-methylimidazole, 1,2-dimethylimidazole, 1-isobutyl-2-methylimidazole, and 1-dimethylaminopropylimidazole; and quaternary ammonium salt compounds, such as tetraalkylammonium halides (e.g., tetramethylammonium chloride), tetraalkylammonium hydroxides (e.g., tetramethylammonium hydroxide salts), tetraalkylammonium organic acid salts (e.g., tetramethylammonium-2-ethylhexanoate, 2-hydroxypropyl trimethylammonium formate, and 2-hydroxypropyl trimethylammonium-2-ethylhexanoate) .

Additives are not particularly limited. Examples include hindered amine-based, benzotriazole-based, and benzophenone-based UV absorbers; perchlorate-based and hydroxylamine-based coloration inhibitors; hindered phenol-based, phosphorus-based, sulfur-based, and hydrazide-based antioxidants; tin-based, zinc-based, and amine-based urethanization catalysts; antifoaming agents, leveling agents, rheology control agents, thixotropy-imparting agents, thickeners, light stabilizers, plasticizers, surfactants, coupling agents, flame retardants, rust inhibitors, fluorescent whitening agents, pigment dispersants, and the like that are commonly used in this technical field.

Pigments are not particularly limited. Examples include organic pigments, such as quinacridone-based, azo-based, and phthalocyanine-based pigments; inorganic pigments, such as titanium oxide, barium sulfate, calcium carbonate, and silica; and other pigments, such as carbon-based pigments, metal foil pigments, and rust-preventive pigments.

Examples of known blocked polyisocyanates include blocked polyisocyanates obtained by reacting polyisocyanates and known blocking agents. Examples of known blocking agents include phenols, such as phenol, thiophenol, methylthiophenol, xylenol, cresol, resorcinol, nitrophenol, and chlorophenol; oximes, such as acetone oxime, methyl ethyl ketone oxime, and cyclohexanone oxime; alcohols, such as methanol, ethanol, n-propyl alcohol, isopropyl alcohol, n-butyl alcohol, isobutyl alcohol, t-butyl alcohol, t-pentanol, ethylene glycol monoethyl ether, ethylene glycol monobutyl ether, diethylene glycol monoethyl ether, propylene glycol monomethyl ether, and benzyl alcohol; pyrazoles, such as 3,5-dimethyl pyrazole and 1,2-pyrazole; triazoles, such as 1,2,4-triazole; halogen-substituted alcohols, such as ethylene chlorohydrin and 1,3-dichloro-2-propanol; lactams, such as $\varepsilon$-caprolactam, $\delta$-valerolactam, $\gamma$-butyrolactam, and $\beta$-propyllactam; active methylene compounds, such as methyl acetoacetate, ethyl acetoacetate, acetylacetone, methyl malonate, and ethyl malonate; and the like. Other examples include amines, imides, mercaptans, imines, ureas, diaryls, and the like.

The thermosetting resin composition of the present invention can be used as paints for automobiles, for buildings, for metal products such as steel furniture, for wooden products such as musical instruments, for mechanical vehicles such as construction machinery, for building materials such as sashes, and for electrical appliances such as office equipment; coating materials for artificial leather, rubber rolls, etc.; inks, adhesives, pressure-sensitive adhesives, sealing materials for electronic components, sealing materials for automobiles, buildings, etc., molding materials for 3D printers, and the like.

Next, the method for curing the thermosetting resin composition of the present invention is explained.

In the method of the present invention, the thermosetting resin composition, which is a mixture of the blocked isocyanate composition and the compound having an isocyanate-reactive group described above, is heated.

The reaction temperature varies depending on the fluoroalcohol-blocked isocyanate compound (BI) and the amidate compound (2) in the blocked isocyanate composition used, but is generally about 60 to 250°C, and preferably about 80 to 200°C. The reaction time is about 30 seconds to 5 hours, and preferably about 1 minute to 60 minutes.

The cured product of the present invention can be produced through the above method for curing the thermosetting resin composition of the present invention.

## Examples

The present invention is described in more detail below with reference to Production Examples and Examples; however, the present invention is not limited to these Examples.

(1) Conditions of Infrared Spectroscopy

Device: FT/IR-6600, produced by JASCO Corporation
Measurement method: total reflection measurement method (crystal: germanium)
Cumulative number: 16

(2) $^1$H-NMR Analysis Conditions

Device: AV400 produced by Bruker Corporation
Frequency: 400 MHz

(3) Curing Temperature Measurement Conditions

Device: Madoka automatic curing time measuring device produced by Cyber Co., Ltd.
Stirring rod: Model number 3JC-5060W
Stirring rate: rotation 100 rpm, revolutions 25 rpm

(4) Measurement of NCO Group Content (%) in Polymeric MDI

The NCO group content (%) as used here refers to the amount of isocyanate groups present in an isocyanate compound expressed as a mass fraction. The NCO group content was measured and calculated according to the following method.

1.6341 g of polymeric MDI (Sumidur 44V20, produced by Sumika Covestro Urethane Co., Ltd.) was placed in a 200-mL conical flask, and 50 mL of a toluene solution of 0.2 mol/L di-n-butylamine was added thereto to dissolve the polymeric MDI. A small amount of bromocresol green was then added to the polymeric MDI solution, and a 0.5 mol/L ethanolic hydrochloric acid solution was added thereto dropwise with a burette. A blank test was also conducted in the same manner, except that polymeric MDI was not used. The amount of the ethanolic hydrochloric acid solution required for the solution in the flask to turn from blue to yellow was 50.17 mL in the blank test and 25.24 mL in the system that used polymeric MDI.

According to the following formula, the NCO group content in the polymeric MDI was calculated to be 32.0%.

NCO group content (%) = [{(titration volume of the ethanolic hydrochloric acid solution in the blank test (mL) - titration volume of the ethanolic hydrochloric acid solution in the sample (mL)} × concentration of the ethanolic hydrochloric acid solution (mol/mL)]/weight of polymeric MDI (g) × 4.202 = [{(50.17 (mL) - 25.24 (mL)} × 0.5 (mol/mL)]/1.6341 (g) × 4.202

(5) Calculation of Effective NCO Group Content (%)

The effective NCO group content (%) as used here is to quantify the amount of blocked isocyanate groups that can be involved in the reaction with isocyanate-reactive groups and that are present in a blocked isocyanate compound after a blocking reaction. The effective NCO group content is expressed as a mass (%) of isocyanate groups and calculated according to the following formula:

$$\text{Effective NCO group content (\%)} = \{(\text{solids content in blocked isocyanate compound (mass (\%))}) \times (\text{mass of isocyanate used in the reaction} \times \text{NCO group content in precursor isocyanate compound (\%)})\}/(\text{mass of resin of blocked isocyanate compound after blocking reaction}).$$

When a solvent etc. was used for dilution, the values of those in the diluted state were used.

(6) Calculation of Solids Content

About 1.5 g of a sample was heated at 110°C for 3 hours, and the solids content (%) in the sample was calculated from the mass before and after heating.

(7) Formulation of Thermosetting Resin Composition

A blocked isocyanate compound, a compound having an isocyanate-reactive group, and an amidate compound were added such that effective NCO group (mol):hydroxyl group (mol):amidate group (mol) = 1.00:0.95:0.05, and methyl isobutyl ketone was added such that solids content in the blocked isocyanate compound (g):solvent (g) = 1.0:1.0. The solvent referred to here includes the solvent used for diluting the blocked isocyanate compound. The effective NCO group (mol) and hydroxyl group (mol) were calculated according to the following formula.

$$\text{Effective NCO group (mol)} = \text{amount of the blocked polyisocyanate used (g)}/\text{effective NCO group content (\%) in the blocked polyisocyanate}/4.202$$

$$\text{Hydroxyl group (mol)} = \text{amount of the polyol used (g)} \times \text{hydroxyl value of the polyol (mgKOH/g)}/56.1$$

Production Example B-1: Synthesis of TFE-blocked HDI biuret

150.0 g (NCO group: 0.81 mol) of HDI biuret (Desmodur N3200A, NCO group content: 22.8 (%), produced by Sumika Covestro Urethane Co., Ltd.), 73.3 g of methyl isobutyl ketone ("MIBK" below) were placed in a 200-mL three-necked reactor purged with nitrogen and heated to 65°C, followed by addition of 1.4 g of triethylamine ("TEA" below). Thereafter, 27.0 g (0.99 mol) of 2,2,2-trifluoroethanol ("TEE" below) and 79.4 g of MIBK were added dropwise to the reactor and stirred at 65°C for 2 hours. Then, the disappearance of the infrared absorption peak of isocyanate group near 2270 $cm^{-1}$ was confirmed by infrared spectroscopic analysis. The obtained reaction solution was concentrated under reduced pressure to remove TEA and most of MIBK, and 59.9 g of MIBK was added, thus obtaining 306.1 g of a MIBK solution of TFE-blocked HDI biuret. The obtained TFE-blocked HDI biuret had a solids content of 76.1%, and an effective NCO group content of 11.2%.

Production Examples B-2 to B-9

Blocked isocyanates were produced in the same manner as in Production Example B-1, except that the components and ratios shown in Table 1 were used. Table 1 shows the results, as well as the reaction temperature and reaction time that were required to confirm the disappearance of the infrared absorption peak of isocyanate group.

Table 1

| | | Production Example B-1 | Production Example B-2 | Production Example B-3 | Production Example B-4 | Production Example B-5 | Production Example B-6 | Production Example B-7 | Production Example B-8 | Production Example B-9 |
|---|---|---|---|---|---|---|---|---|---|---|
| Formulation used for reaction | Isocyanate | HDI biuret(1) | HDI biuret(1) | HDI biuret(1) | HDI biuret(1) | HDI isocyanurate (2) | HDI isocyanurate (3) | Polymeric MDI(4) | HDI biuret(1) | HDI biuret(1) |
| | Weight (g) | 150.0 | 30.2 | 18.0 | 30.1 | 60.1 | 70.1 | 40.1 | 60.0 | 60.0 |
| | NCO group (mol) | 0.81 | 0.16 | 0.10 | 0.16 | 0.31 | 0.20 | 0.31 | 0.33 | 0.33 |
| | Blocking agent | TFE(5) | DFE(6) | HpFB(7) | HFIP(8) | TFE(5) | TFE(5) | TFE(5) | MEKO(9) | EtOH(10) |
| | Weight (g) | 989 | 16 | 23.6 | 32.9 | 37.7 | 36.9 | 36.8 | 29.0 | 19.0 |
| | Substance amount (mol) | 0.99 | 0.20 | 0.12 | 0.20 | 0.38 | 0.37 | 0.37 | 0.33 | 0.41 |
| | Solvent | MIBK | MIBK | MIBK | MIBK | MIBK | n-Butyl acetate | MIBK | MIBK | MIBK |
| | Weight (g) | 148.3 | 30.2 | 18.0 | 30.1 | 60.4 | 28.0 | 40.8 | 59.7 | 60.0 |
| | Triethylamine (g) | 1.5 | 0.3 | 0.2 | 0.3 | 0.6 | 0.5 | 0.4 | 0.6 | 0.6 |
| Reaction conditions | Temperature (°C) | 65 | 65 | 65 | 55 | 65 | 70 | 60 | 65 | 75 |
| | Time (hr) | 2 | 6 | 6 | 1.5 | 2 | 7.5 | 2.5 | 2 | 6.5 |
| Solvent added after concentration | | MIBK | MIBK | MIBK | MIBK | MIBK | n-Butyl acetate | MIBK | MIBK | MIBK |
| Weight (g) | | 59.9 | 11.8 | 9.9 | 11.9 | 20.0 | 16.7 | 78.7 | 17.4 | 17.6 |
| Blocked isocyanate | | HDI biuret | HDI biuret | HDI biuret | HDI biuret | HDI isocyanurate | HDI isocyanurate | Polymeric MDI | HDI biuret | HDI biuret |
| | | TFE-blocked form | DFE-blocked form | HpFB-blocked form | HFIP-blocked form | TFE-blocked form | TFE-blocked form | TFE-blocked form | MEKO-blocked form | EtOH-blocked form |
| Solids content (%) | | 76.1 | 74.7 | 74.6 | 74.3 | 71.2 | 72.5 | 41.5 | 74.7 | 75.7 |

(continued)

| | Production Example B-1 | Production Example B-2 | Production Example B-3 | Production Example B-4 | Production Example B-5 | Production Example B-6 | Production Example B-7 | Production Example B-8 | Production Example B-9 |
|---|---|---|---|---|---|---|---|---|---|
| Effective NCO group content (%) | 11.2 | 12.0 | 8.4 | 8.9 | 10.2 | 8.9 | 7.7 | 11.6 | 14.0 |

(1) Sumidur N3200A, produced by Sumika Covestro Urethane Co., Ltd., solids content: 100%, NCO group content: 22.8%
(2) Desmodur N3300, produced by Sumika Covestro Urethane Co., Ltd., solids content: 100%, NCO group content: 21.9%
(3) Desmodur Z4470BA, produced by Sumika Covestro Urethane Co., Ltd., solids content: 69.7%, NCO group content: 11.9%
(4) Sumidur 44V20, produced by Sumika Covestro Urethane Co., Ltd., solids content: 100%, NCO group content: 32.0%
(5) 2,2,2-Trifluoroethanol
(6) 2,2-Difluoroethanol
(7) 2,2,3,3,4,4,4-Heptafluoro-1-butanol
(8) 1,1,1,3,3,3-Hexafluoro-2-propanol
(9) Ethyl methyl ketoxime
(10) Ethanol

Production Example A-1: Synthesis of DOIm_PI

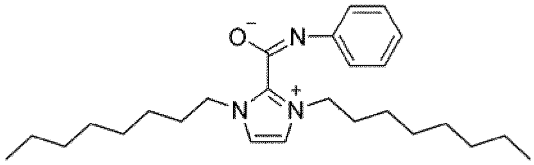

Step 1

1049.9 g (8.12 mol) of n-octylamine was placed in a 3-L four-necked reactor purged with nitrogen and heated to 80°C. Subsequently, a mixture of 366.0 g (6.09 mol) of acetic acid and 290.4 g of 42 wt% formalin aqueous solution (formaldehyde pure content: 4.06 mol) was added dropwise to the reactor over 1 hour. The solution after dropwise addition was stirred for 30 minutes and cooled to 40°C. To the solution cooled to 40°C, 581.0 g of 41 wt% glyoxal aqueous solution (glyoxal pure content: 4.06 mol) was added dropwise over 30 minutes and stirred for 5 hours. After stirring, the obtained reaction solution was concentrated under reduced pressure to give 1582.0 g of a reaction product containing 1,3-di-n-octylimidazolium acetate. The results of $^{1}$H-NMR analysis with tetralin added as an internal standard substance revealed that the pure content of 1,3-di-n-octylimidazolium acetate was 1224.9 g (3.47 mol, yield: 85.5%).

Step 2

1070.1 g (pure content: 828.6 g, 2.35 mol) of the reaction product obtained in step 1, 1069.5 g of toluene, and 756.0 g (8.39 mol) of dimethyl carbonate were placed in a 5-L pressure-resistant container, followed by purging with nitrogen. The mixture was then stirred at 120°C for 15 hours. After stirring, the obtained reaction mixture was concentrated under reduced pressure to 1043.5 g, to which toluene was added to give 2058.6 g of a toluene solution.

Step 3

1700.2 g of the toluene solution obtained in step 2 and 807.4 g of toluene were placed in a 3-L four-necked reactor purged with nitrogen and heated under reflux. To the reaction liquid heated under reflux, 229.7 g (1.93 mol) of phenyl isocyanate was added dropwise over 2 hours and stirred for 10 hours. After stirring, the obtained reaction mixture was concentrated to 1433.1 g, and 761.0 g of heptane was added. The resulting mixture was heated to 50°C to dissolve all of the solids to obtain a mixed solution. The obtained mixed solution was then cooled from 50°C to 30°C to precipitate crystals, followed by stirring at 30°C for 1 hour. Subsequently, the mixed solution was cooled to 10°C over a period of 2 hours at 10°C per hour and stirred at 10°C for another 1 hour to obtain a slurry liquid. The obtained slurry liquid was filtered to give 426.8 g (1.04 mol; overall yield from step 2: 53.2%) of a compound represented by the above formula (DOIm_PI) as a pale yellow solid. The $^{1}$H-NMR analysis results of DOIm_PI are shown below. $^{1}$H-NMR(DMSO-d6)$\delta$(ppm)=9.32 (s, 1H), 7.80 (s, 2H), 4.17 (t, J=9.6 Hz, 4H), 1.78 (m, 4H), 1.63 (s, 3H), 1.23 (m, 20H), 0.85 (t, J=6.4 Hz, 6H)

Production Example A-2: Synthesis of DtBIm_PI

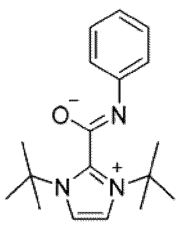

Step 1

100.0 g (1.36 mol) of tert-butylamine was placed in a 300-mL four-necked reactor purged with nitrogen and

heated to 40°C. Subsequently, a mixture of 62.1 g (1.03 mol) of acetic acid and 50.8 g of 40 wt% formalin aqueous solution (formaldehyde pure content: 0.68 mol) was added dropwise to the reactor over 1 hour and stirred for 30 minutes. To the stirred solution, 99.0 g of 40 wt% glyoxal aqueous solution (glyoxal pure content: 0.68 mol) was added dropwise over 30 minutes and stirred for 5 hours. After stirring, the obtained reaction solution was concentrated under reduced pressure, and 100.0 g of $H_2O$ and 100.0 g of toluene were added to carry out a liquid separation operation. The aqueous layer obtained after liquid separation was concentrated under reduced pressure to give 123.2 g of a reaction product containing 1,3-di-tert-butylimidazolium acetate.

Step 2

40.0 g of the reaction product containing 1,3-di-tert-butylimidazolium acetate obtained in step 1 and 52.6 g (0.58 mol) of dimethyl carbonate were placed in a 180-mL pressure-resistant container, followed by purging with nitrogen. The mixture was then stirred at 120°C for 6 hours. After stirring, the obtained reaction mixture was concentrated under reduced pressure to give a black solid. The obtained black solid was washed with 100.0 g of acetone, thus obtaining 38.6 g of a white solid.

Step 3

10.0 g of the white solid obtained in step 2 and 40.0 g of chlorobenzene were placed in a 100-mL three-necked reactor purged with nitrogen and heated under reflux. After heating under reflux, a mixture of 5.9 g of phenyl isocyanate and 10.0 g of chlorobenzene was added dropwise to the reactor over 10 minutes and stirred for 1 hour. After stirring, the obtained reaction mixture was filtered, and the filter residue was washed with 50.0 g of heptane to give 7.84 g (0.017 mmol, overall yield from step 1: 30.0%) of a composition comprising a compound represented by the above formula (DtBIm_PI). The $^1$H-NMR analysis results of DtBIm_PI are shown below.

$^1$H-NMR($CDCl_3$)δ(ppm)=7.56 (d, J=6.6 Hz, 2H), 7.31 (t, J=6.6 Hz, 2H), 7.07 (s, 2H) 6.93 (t, J=7.2 Hz, 1H), 1.87 (s, 18H)

Production Example A-3: Synthesis of DtOIm_PI

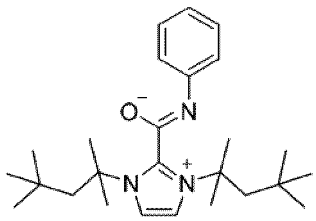

Step 1

6.97 g (0.116 mol) of acetic acid, 5.80 g of 40 wt% formalin aqueous solution (formaldehyde pure content: 0.077 mol), and 11.2 g of 40 wt% glyoxal aqueous solution (glyoxal pure content: 0.077 mol) were placed in a 100-ml three-necked reactor purged with nitrogen and heated to 50°C. Then, 20.00 g (0.154 mol) of 1,1,3,3-tetramethylbutylamine was added dropwise to the mixture in the reactor over 2 hours and stirred for 2 hours. After stirring, the obtained reaction solution was concentrated under reduced pressure to give 24.8 g of a reaction product containing 1,3-bis(1,1,3,3-tetramethylbutyl)imidazolium acetate.

Step 2

24.8 g of the reaction product containing 1,3-bis(1,1,3,3-tetramethylbutyl)imidazolium acetate obtained in step 1, 27.0 g (0.297 mol) of dimethyl carbonate, and 30.0 g of toluene were placed in a 180-mL pressure-resistant container, followed by purging with nitrogen. The mixture in the pressure-resistant container was then stirred at 120°C for 6 hours. After stirring, the obtained reaction mixture was concentrated under reduced pressure to give 20.0 g of a dark brown solid.

Step 3

10.4 g of the dark brown solid obtained in step 2 and 40.0 g of chlorobenzene were placed in a 100-mL three-necked reactor purged with nitrogen and heated under reflux. After heating under reflux, the mixture was cooled to 60°C, and a mixture of 3.9 g (0.032 mol) of phenyl isocyanate and 12.2 g of chlorobenzene was added thereto dropwise over 10 minutes and stirred at 60°C for 1 hour. After stirring, the obtained reaction mixture was cooled to room temperature, and 50.0 g of heptane was added. Then, the resulting mixture was filtered, and the filter residue was washed with 50.0 g of heptane to give 7.55 g (0.014 mmol, overall yield from step 1: 36.0%) of a composition comprising a compound represented by the above formula (DtOIm_PI). The $^1$H-NMR analysis results of DtOIm_PI are shown below.

$^1$H-NMR($CDCl_3$)δ(ppm)=7.59 (d, J=9.2 Hz, 2H), 7.30 (t, J=7.6 Hz, 2H), 7.08 (s, 2H) 6.93 (t, J=7.2 Hz, 1H), 2.40 (m, 4H), 1.87 (m, 12H), 0.99 (s, 18H)

Production Example A-4: Synthesis of DtOIm_crMDI

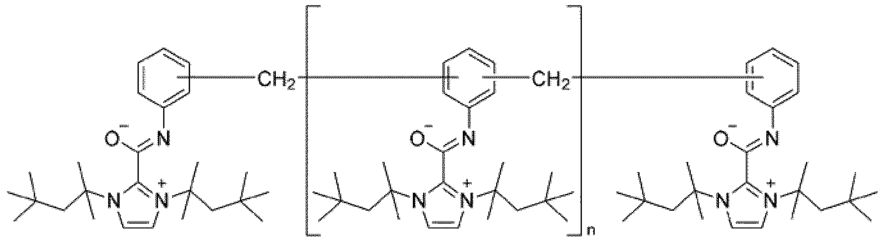

5.0 g of the dark brown solid obtained in step 2 of Production Example A-3 and 20.0 g of chlorobenzene were placed in a 100-mL three-necked reactor purged with nitrogen and heated under reflux. After heating under refluxing, a mixture of 1.52 g (NCO group: 0.012 mol) of polymeric MDI (Sumidur 44V20, solids content: 100%, NCO group content: 32.0%, produced by Sumika Covestro Urethane Co., Ltd.) and 5.0 g of chlorobenzene was added dropwise to the reactor over 10 minutes and stirred for 1 hour. After stirring, the obtained reaction mixture was added dropwise to 50.0 g of heptane and filtered, and the filter residue was washed with 50.0 g of heptane to give 4.28 g (0.014 mmol, overall yield from step 1: 52.0%) of a composition comprising a compound represented by the above formula (DtOIm_crMDI). The $^1$H-NMR analysis results of DtOIm_crMDI are shown below. $^1$H-NMR($CDCl_3$)δ(ppm)=7.52-7.48 (m), 7.35-7.30 (m), 7.13-7.11 (m) 7.04-7.00 (m), 1.87 (m), 1.32 (m), 0.93 (m)

Production Example A-5: Synthesis of OMIm_PI

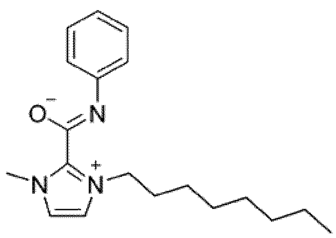

Step 1

25.0 g (139 mmol) of 1-octylimidazole, 16.7 g (185 mmol) of dimethyl carbonate, and 25.1 g of methanol were placed in a 180-mL autoclave purged with nitrogen and stirred at 125°C for 29 hours. After stirring, the reaction mixture was cooled to room temperature. To the cooled reaction mixture was added 8.5 g (94 mmol) of dimethyl carbonate, and the mixture was stirred at 130°C for another 3 hours. The obtained reaction mixture was cooled to 25°C to give 44.0 g of a methanol solution of 1-octyl-3-methylimidazolium-2-carboxylate (abbreviated below as "OMIm-$CO_2$") (pure content: 33.0 g, 139 mmol, yield: 99%).

Step 2

4.0 g (pure content: 13 mmol) of the methanol solution of OMIm-$CO_2$ obtained in step 1, 1.5 g (13 mmol) of phenyl isocyanate, and 100 mL of toluene were placed in a 200-mL test tube purged with nitrogen, and the obtained mixture was stirred at an internal temperature of 110°C for 3 hours. After concentration under reduced pressure, 3.3 g of a compound represented by the above formula (OMIm-PI) was obtained. Furthermore, high-purity OMIm-PI was obtained by recrystallization with butyl acetate. The $^1$H-NMR analysis results of the compound represented by the above formula are shown below. $^1$H-NMR($CD_3OD$)$\delta$(ppm)=7.51 (s, 1H), 7.45-7.33 (m, 6H), 4.37 (t, J=7.4 Hz, 2H), 3.97 (s, 3H), 1.91-1.86 (m, 2H), 1.35-1.27 (m, 10H), 0.88 (t, J=6.8 Hz, 3H)

Example 1

The TFE-blocked HDI biuret obtained in Production Example B-1, a polyester polyol (P-510, produced by Kuraray Co., Ltd.), and OMIm_PI obtained in Production Example A-5 were added such that the formulation of a thermosetting resin composition satisfied that effective NCO group (mol):hydroxyl group (mol):amidate group (mol) = 1.00:0.95:0.05. Further, MIBK was added such that the amount of solvent was 1.0 times by weight relative to the blocked isocyanate compound. The mixture was then stirred for 30 minutes, thus preparing a thermosetting resin composition.

About 0.6 mL of the prepared thermosetting resin composition was poured onto the hot plate of the automatic curing time measuring device that had been heated to 90°C, and stirring was performed. During this procedure, the curing time was measured and evaluated, taking the time between the stirring torque immediately after the start of stirring of less than 1% (0.04 mN•m) and the stirring torque exceeding 20% (0.86 mN•m) as the curing time. Tables 2 to 4 show the results.

Examples 2 to 12 and Comparative Examples 1 to 6

Thermosetting resin compositions were prepared in the same manner as in Example 1, except that the polyol, blocked isocyanate, and catalyst were changed to those shown in Tables 2 to 4, and the curing time was measured and evaluated. Tables 2 to 4 show the results.

In Tables 1 to 4, TFE represents 2,2,2-trifluoroethanol, DFE represents 2,2-difluoroethanol, HFIP represents 1,1,1,3,3,3-hexafluoro-2-propanol, HpFB represents 2,2,3,3,4,4,4-heptafluoro-1-butanol, MEKO represents ethyl methyl ketoxime, and EtOH represents ethanol. Me represents a methyl group, t-Bu represents a tert-butyl group, n-Oct represents an n-octyl group, and t-Oct represents a 1,1,3,3-tetramethylbutyl group.

In Tables 2 to 4, G, J, and B denote the groups corresponding to the symbols used in formula (2).

Table 2

| | | Ex. 1 | Ex. 2 | Ex. 3 | Ex. 4 | Ex. 5 | Comp. Ex. 1 | Comp. Ex. 2 |
|---|---|---|---|---|---|---|---|---|
| Polyol | | P-510 | P-510 | P-510 | P-510 | P-510 | P-510 | P-510 |
| Blocked isocyanate | Production Example | B-1 | B-1 | B-1 | B-1 | B-1 | B-1 | B-1 |
| | Isocyanate | HDI biuret | HDI biuret | HDI biuret | HDI biuret | HDI biuret | HDI biuret | HDI biuret |
| | Blocking agent | TFE | TFE | TFE | TFE | TFE | TFE | TFE |
| Catalyst | | MOIm_PI | DOIm_PI | DtBIm_PI | DtOIm_PI | DtOIm_crMDI | None | DBTDL |
| G | | Me | nOct | tBu | tOct | tOct | - | - |
| J | | nOct | nOct | tBu | tOct | tOct | - | - |
| B | | Ph | Ph | Ph | Ph | Polymethylene polyphenyl group | - | - |
| Curing time at 90°C | | B | A | S+ | S+ | S+ | C | C |

Table 3

| | | Ex. 6 | Ex. 7 | Ex. 8 | Ex. 9 | Comp. Ex. 3 | Comp. Ex. 4 | Comp. Ex. 5 |
|---|---|---|---|---|---|---|---|---|
| Polyol | | P-510 | P-510 | P-510 | P-510 | P-510 | P-510 | P-510 |
| Blocked isocyanate | Production Example | B-2 | B-4 | B-4 | B-3 | B-8 | B-8 | B-8 |
| | Isocyanate | HDI biuret | HDI biuret | HDI biuret | HDI biuret | HDI biuret | HDI biuret | HDI biuret |
| | Blocking agent | DFE | HFIP | HFIP | HpFB | MEKO | MEKO | MEKO |
| Catalyst | | DtOIm_PI | DOIm_PI | DtOIm_PI | DtOIm_PI | DBTDL | MOIm_PI | DOIm_PI |
| | G | tOct | nOct | tOct | tOct | - | Me | nOct |
| | J | tOct | nOct | tOct | tOct | - | nOct | nOct |
| | B | Ph | Ph | Ph | Ph | - | Ph | Ph |
| Curing time at 90°C | | A$^+$ | B$^+$ | A | A$^+$ | C | C | C |

Table 4

| | | Comp. Ex. 6 | Ex. 10 | Ex. 11 | Ex. 12 |
|---|---|---|---|---|---|
| Polyol | | P-510 | P-510 | P-510 | P-510 |
| Blocked isocyanate | Production Example | B-9 | B-5 | B-6 | B-7 |
| | Isocyanate | HDI biuret | HDI isocyanurate | HDI isocyanurate | Polymeric MDI |
| | Blocking agent | EtOH | TFE | TFE | TFE |
| Catalyst | | DOIm_PI | DtOIm_PI | DtOIm_PI | DtOIm_PI |
| | G | nOct | tOct | tOct | tOct |
| | J | nOct | tOct | tOct | tOct |
| | B | Ph | Ph | Ph | Ph |
| Curing time at 90°C | | C | S | S | S$^+$ |

Curing time at 90°C: S$^+$: 12 min or less, S: 20 min or less, A$^+$: 30 min or less, A: 35 min or less, B$^+$: 40 min or less, B: 50 min or less, and C: exceeding 50 min.

The results of Examples 1 to 12 and Comparative Examples 1 to 6 revealed that the resin compositions comprising the blocked isocyanate compositions of the present invention were cured within a short period of time even at low temperatures and thus had excellent low-temperature curing properties. Of these, the results also revealed that the use of DtBIm_PI and DtOIm_PI, which are amidate compounds with bulky substituents, i.e., tertiary alkyl groups

such as a tert-butyl group and a 1,1,3,3-tetramethylbutyl group, achieved satisfactory low-temperature curing properties. In particular, combinations with blocked polyisocyanates blocked with primary fluoroalcohol compounds, such as 2,2,2-trifluoroethanol and 2,2-difluoroethanol, achieved excellent low-temperature curing properties.

## Claims

1. A blocked isocyanate composition comprising a blocked isocyanate compound in which an isocyanate group of an isocyanate compound is blocked with a fluoroalcohol compound, and at least one amidate compound represented by the following Formula (2):

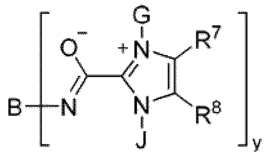 (2)

wherein B represents a substituted or unsubstituted hydrocarbon group; G and J are the same or different, and each represents a $C_1$-$C_{80}$ hydrocarbon group that may be substituted with a heteroatom; $R^7$ and $R^8$ are the same or different, and each represents a hydrogen atom or a $C_1$-$C_{20}$ hydrocarbon group that may be substituted with a heteroatom; G and $R^7$, or $R^8$ and J may form a ring structure together with the nitrogen and carbon atoms to which they are bonded; $R^7$ and $R^8$ may form a ring structure together with the carbon atoms to which they are bonded; and y is an integer of 1 or more and 20 or less.

2. The blocked isocyanate composition according to claim 1, wherein the isocyanate compound is at least one polyisocyanate selected from the group consisting of aliphatic polyisocyanates, alicyclic polyisocyanates, aromatic polyisocyanates, and aromatic aliphatic polyisocyanates; or a modified isocyanate formed from at least one member selected from the group consisting of aliphatic polyisocyanates, alicyclic polyisocyanates, aromatic polyisocyanates, and aromatic aliphatic polyisocyanates.

3. The blocked isocyanate composition according to claim 1, wherein the isocyanate compound is represented by the following Formula (3):

$$A\!-\![NCO]_x \quad (3)$$

wherein A represents a residue obtained by removing an isocyanate group from at least one polyisocyanate selected from the group consisting of aliphatic polyisocyanates, alicyclic polyisocyanates, aromatic polyisocyanates, and aromatic aliphatic polyisocyanates, or a residue obtained by removing an isocyanate group from a modified isocyanate formed from at least one member selected from the group consisting of aliphatic polyisocyanates, alicyclic polyisocyanates, aromatic polyisocyanates, and aromatic aliphatic polyisocyanates; and x is an integer of 2 or more and 20 or less.

4. The blocked isocyanate composition according to any one of claims 1 to 3, wherein the fluoroalcohol compound is represented by Formula (4):

HO-L (4)

wherein L represents a $C_1$-$C_{40}$ fluorine-containing alkyl group.

5. The blocked isocyanate composition according to any one of claims 1 to 3, wherein the fluoroalcohol compound is represented by Formula (4a):

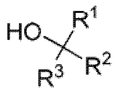 (4a)

wherein $R^1$ and $R^2$ are the same or different, and each represents a hydrogen atom or a $C_1$-$C_8$ fluorine-containing

alkyl group; and $R^3$ represents a $C_{1-}C_{20}$ fluorine-containing alkyl group.

**6.** The blocked isocyanate composition according to claim 5, wherein $R^1$ and $R^2$ are the same or different, and each represents a hydrogen atom or a $C_{1-}C_2$ fluorine-containing alkyl group.

**7.** The blocked isocyanate composition according to any one of claims 1 to 6, wherein the amidate compound represented by Formula (2) is an amidate compound represented by Formula (2a):

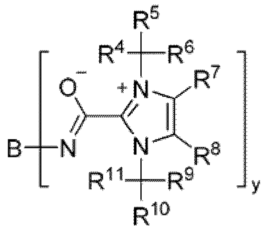
(2a)

wherein B, $R^7$, $R^8$, and y are each as defined above; $R^4$, $R^5$, $R^6$, $R^9$, $R^{10}$, and $R^{11}$ are the same or different, and each represents a $C_{1-}C_{20}$ hydrocarbon group that may be substituted with a heteroatom; $R^4$ and $R^5$, $R^5$ and $R^6$, $R^7$ and $R^8$, $R^9$ and $R^{10}$, or $R^{10}$ and $R^{11}$ may form a ring structure together with the carbon atoms to which they are bonded; and the group represented by $CR^4R^5R^6$ or the group represented by $CR^9R^{10}R^{11}$ may be an adamantyl group.

**8.** A thermosetting resin composition comprising the blocked isocyanate composition according to any one of claims 1 to 7 and a compound having an isocyanate-reactive group.

**9.** The thermosetting resin composition according to claim 8, wherein the compound having an isocyanate-reactive group is a polyol compound.

**10.** A cured product obtained by curing the thermosetting resin composition according to claim 8 or 9.

**11.** A method for producing a cured product, comprising curing the thermosetting resin composition according to claim 8 or 9 by heating.

**12.** An amidate compound represented by the following Formula (2a):

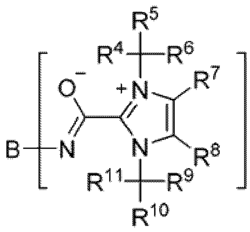
(2a)

wherein B represents a substituted or unsubstituted hydrocarbon group; $R^4$, $R^5$, $R^6$, $R^9$, $R^{10}$, and $R^{11}$ are the same or different, and each represents a $C_{1-}C_{20}$ hydrocarbon group that may be substituted with a heteroatom, or $R^4$ and $R^5$, $R^5$ and $R^6$, $R^9$ and $R^{10}$, or $R^{10}$ and $R^{11}$ may form a ring structure together with the carbon atoms to which they are bonded; $R^7$ and $R^8$ are the same or different, and each represents a hydrogen atom or a $C_{2-}C_{20}$ hydrocarbon group that may be substituted with a heteroatom, or $R^7$ and $R^8$ may form a ring structure together with the carbon atoms to which they are bonded; y is an integer of 1 or more and 20 or less; and the group represented by $CR^4R^5R^6$ or the group represented by $CR^9R^{10}R^{11}$ may be an adamantyl group.

**13.** The amidate compound according to claim 12, wherein $R^4$, $R^5$, $R^9$, and $R^{10}$ are the same or different, and each represents a $C_{1-}C_4$ hydrocarbon group; and $R^6$ and $R^{11}$ are the same or different, and each represents a $C_{1-}C_{12}$ hydrocarbon group.

**14.** The amidate compound according to claim 12, wherein the compound represented by Formula (2a) is any of compounds represented by the following formulas:

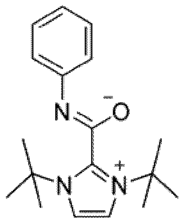,
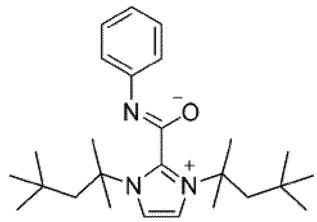,

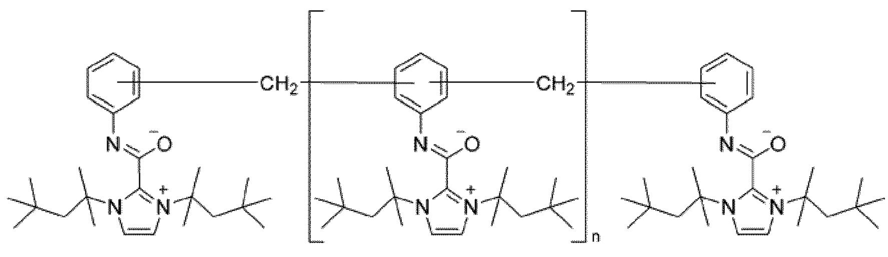,
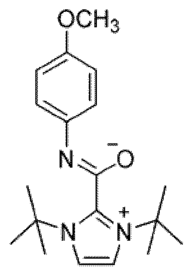,

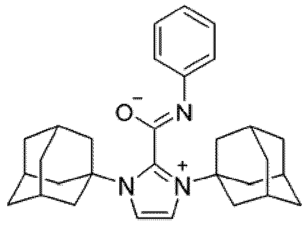

wherein n is 0 or an integer of 1 to 4.

**15.** A blocking agent dissociation catalyst for blocked isocyanates comprising the amidate compound according to any one of claims 12 to 14.

**INTERNATIONAL SEARCH REPORT**

International application No. PCT/JP2021/016895

A. CLASSIFICATION OF SUBJECT MATTER
Int.Cl. C08G18/20(2006.01)i, C08G18/80(2006.01)i
FI: C08G18/20, C08G18/80087

According to International Patent Classification (IPC) or to both national classification and IPC

B. FIELDS SEARCHED

Minimum documentation searched (classification system followed by classification symbols)
Int.Cl. C08G18/20, C08G18/80

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y<br>A | WO 2019/065953 A1 (KOEI CHEM CO., LTD.) 04 April 2019 (2019-04-04), claims 1, 8, paragraph [0008], example 4 | 1-6, 8-11<br>7, 12-15 |
| Y<br>A | JP 6-157450 A (HITACHI MAXELL LTD.) 03 June 1994 (1994-06-03), claim 1, paragraph [0008], examples 1, 2 | 1-6, 8-11<br>7, 12-15 |
| A | WO 2019/066029 A1 (KANSAI PAINT CO., LTD.) 04 April 2019 (2019-04-04), entire text | 1-15 |
| A | WO 2020/067431 A1 (KOEI CHEM CO., LTD.) 02 April 2020 (2020-04-02), entire text | 1-15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 June 2021 | 15 June 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/016895

| | | |
|---|---|---|
| WO 2019/065953 A1 | 04 April 2019 | US 2020/0216600 A1<br>claims 1, 8, paragraph [0009], example 4<br>EP 3689931 A1<br>CN 111094375 A<br>KR 10-2020-0060367 A<br>TW 201920118 A |
| JP 6-157450 A | 03 June 1994 | (Family: none) |
| WO 2019/066029 A1 | 04 April 2019 | EP 3689979 A1<br>entire text<br>CN 111094471 A |
| WO 2020/067431 A1 | 02 April 2020 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019065953 A1
- JP S5734107 A
- JP S61275311 A


### Non-patent literature cited in the description


- *Structural Chemistry,* 2013, vol. 24, 2059-2068